# EUROPÄISCHE PATENTSCHRIFT

(11) **EP 0 516 257 B1**
(45) Veröffentlichungstag und Bekanntmachung des Hinweises auf die Patenterteilung: **05.08.1998**
(21) Anmeldenummer: 92250129.1
(22) Anmeldetag: 25.05.1992
(51) Int. Cl.: C07D 307/80, C07D 307/81, C07D 333/56, C07D 333/58, A61K 31/38, A61K 31/34

(54) **2-Phenylbenzo[b]furane und -thiophene, Verfahren zu deren Herstellung und diese enthaltende pharmazeutische Präparate**
Phenylbenzo[b]furans and -thiophenes, procedures for their preparation, and pharmaceutical compositions containing the same
Phénylbenzo[b]furannes et -thiophènes, procédés de leur préparation et compositions pharmaceutiques les contenant

(30) Priorität: 25.05.1991 DE 4117512
(43) Veröffentlichungstag der Anmeldung: 02.12.1992
(73) Patentinhaber: SCHERING AKTIENGESELLSCHAFT, 13342 Berlin (DE)
(72) Erfinder: Von Angerer, Erwin, Prof. Dr., W-8401 Grasslfing (DE); Erber, Sebastian, Dr., W-1000 Berlin 27 (DE); Schneider, Martin, Dr., W-1000 Berlin 28 (DE)

(56) Entgegenhaltungen:
- EP-A- 0 062 503
- EP-A- 0 062 504
- EP-A- 0 062 505
- EP-A- 0 124 369
- JOURNAL OF MEDICINAL CHEMISTRY. Bd. 32, 1989, WASHINGTON US Seiten 1700 - 1707; N. DURANI ET AL.: 'Structure-Activity Relationship of Antiestrogens: A Study Using Triarylbutenone, Benzofuran, and Triarylfuran Analogues as Models for Triarylethylenes and Triarylpropenones'
- JOURNAL OF MEDICINAL CHEMISTRY. Bd. 27, 1984, WASHINGTON US Seiten 1057 - 1066; CH. D. JONES ET AL.: 'Antiestrogenes. 2. Structure-Activity Studies in a Series of 3-Aroyl-2-arylbenzo[b]thiophene Derivatives Leading to [6-Hydroxy-2-(4- hydroxyphenyl)benzo[b ]thien-3-yl][4-[2-(1-piperidinyl)ethoxy]-phenyl] methanone Hydrochloride (LY156758), a Remarkably Effective Estrogen Antagonist with Only Minimal...'
- CHIMICA THERAPEUTICA. Bd. 4, 1973, FR Seiten 398 - 411; C. GOLDENBERG ET AL.: 'Recherches dans la série des benzofurannes. LII. Synthèse de dérivés d'hydroxy-5 ou -6 p-hydroxyphényl-2 ou -3 benzofurannes'
- CHEMICAL ABSTRACTS, vol. 113, no. 5, 30. Juli 1990, Columbus, Ohio, US; abstract no. 34694W, H. NAITO ET AL.: 'Benzofurans as medicinal bactericides and fungicides' Seite 48 ;Spalte 1 ;
- CHEMICAL ABSTRACTS, vol. 116, no. 12, 30. März 1992, Columbus, Ohio, US;
- abstract no. 120397W, S. ERBER ET AL.: '2-Phenylbenzo[b]furans: relationship between structure, estrogen receptor affinity and cytostatic activity against mammary tumor cells' Seite 18 ;Spalte 2 ;
- CHEMICAL ABSTRACTS, vol. 117, no. 3, 20. Juli 1992, Columbus, Ohio, US; abstract no. 20038F, E. VON ANGERER ET AL.: '3-Alkyl-2- phenylbenzo[b]thiophenes: nonsteroidal estrogen antagonists with mammary tumor inhibiting activity.' Seite 39 ;Spalte 1 ;

## Beschreibung

Die Erfindung betrifft 2-Phenylbenzo[b]furane und -thiophene, Verfahren zu deren Herstellung und diese enthaltende pharmazeutische Präparate.

Die neuen Furane und Thiophene werden durch die allgemeine Formel I gekennzeichnet 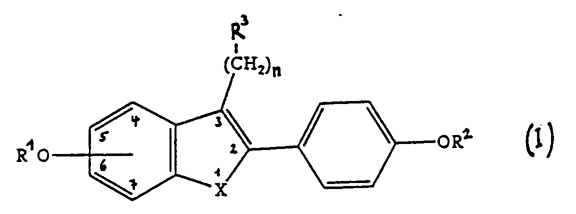
worin R¹ und R² unabhängig voneinander ein Wasserstoffatom, eine Alkylgruppe mit 1 bis 10 Kohlenstoffatomen, eine Benzylgruppe, eine Gruppe -C(O)R⁴, wobei R⁴ eine Alkyl- oder Alkyloxygruppe mit 1 bis 10 Kohlenstoffatomen oder ein Phenylrest ist, oder eine Carbamoylgruppe -C(O)NR⁵R⁶, wobei R⁵ und R⁶ unabhängig voneinander ein Wasserstoffatom oder ein Alkylgruppe mit 1 bis 10 Kohlenstoffatomen ist,
X ein Sauerstoff- oder Schwefelatom und n eine ganze Zahl von 4 bis 12, wenn R³ eine Aminogruppe -NR⁷R⁸ ist, wobei R⁷ und R⁸ unabhängig voneinander für ein Wasserstoffatom oder eine Alkylgruppe mit 1 bis 10 Kohlenstoffatomen oder R⁷-R⁸ gemeinsam für eine Alkylengruppe -(CH₂)ₘ-, wobei m 2, 3, 4, 5 oder 6 ist, oder die Gruppe -(CH₂)₂O(CH₂)₂- stehen, oder
X ein Sauerstoff- oder Schwefelatom und n eine ganze Zahl von 0 bis 12 wenn R³ ein Wasserstoffatom ist, wobei n nicht 0 sein kann, wenn X ein Schwefelatom ist und wobei n nicht 0 oder 1 sein kann, wenn X ein Sauerstoffatom ist,
bedeuten.

Als Alkanoylgruppen -C(O)R⁴ kommen Reste von organischen Carbonsäuren mit 1 bis 10 Kohlenstoffatomen infrage, die gesättigt oder ungesättigt sein können. Sie leiten sich ab von aliphatischen, cycloaliphatischen, aliphatisch-cycloaliphatischen, cycloaliphatisch-aliphatischen und aromatischen Monocarbonsäuren. Die Anzahl der Kohlenstoffatome im Ring variiert von 3 bis 7. Bevorzugt werden als Reste R¹ und R² die Alkanoyloxygruppen der Essig-, Propion-, Butter-, Isobutter-, Pivalin-, Capron-, Acryl-, Croton-, Heptyl-, Capryl-, Pelargon-, Decan-, 3-Cyclopentylpropion- und Benzoesäure.

Der Rest R¹ kann sich in den Positionen 4, 5, 6 und 7 des Bicyclus befinden, besonders geeignet sind die 5- und 6-Position.

Alkylgruppen mit 1 bis 10 Kohlenstoffatomen und Cycloalkylgruppen mit 3 bis 7 Kohlenstoffatomen kommen als Reste R⁵ und R⁶ infrage.

Als Alkylgruppen R⁵, R⁶, R⁷ und/oder R⁸ sind die Reste Methyl, Ethyl, Propyl, Butyl, Pentyl, Hexyl, Heptyl, Octyl, Nonyl und Decanyl geeignet. Als Cycloalkylgruppen sind besonders die Reste Cyclopentyl und Cyclohexyl zu nennen.

Als Beispiel einer Aralkylgruppe für die Reste R⁴, R⁵ und/oder R⁶ ist insbesondere die Benzylgruppe zu nennen.

Die Reste R⁷ und R⁸ können gleich oder verschieden oder Bestandteile eines gemeinsamen Ringes sein. Beim Vorliegen eines Ringes kann dieser neben dem Stickstoffatom zusätzlich ein Sauerstoffatom enthalten. Als solche Reste sind insbesondere geeignet die Kombination Wasserstoff/Methyl (abgeleitet von Methylamino), Wasserstoff/Wasserstoff (abgeleitet von Amino), Methyl/Methyl (abgeleitet von Dimethylamino), sowie der -(CH₂)₄-Rest (abgeleitet von Pyrrolidino), der -(CH₂)₅-Rest (abgeleitet von Piperidino) und der -(CH₂)₂-O-(CH₂)₂-Rest (abgeleitet von Morpholino).

Die Erfindung betrifft insbesondere folgende Verbindungen:
3-Ethyl-5-hydroxy-2-(4-hydroxyphenyl)benzo[b]furan
3-Ethyl-6-hydroxy-2-(4-hydroxyphenyl)benzo[b]furan
5-Hydroxy-2-(4-hydroxyphenyl)-3-propylbenzo[b]furan
6-Hydroxy-2-(4-hydroxyphenyl)-3-propylbenzo[b]furan
3-Butyl-5-hydroxy-2-(4-hydroxyphenyl)benzo[b]furan
3-Butyl-6-hydroxy-2-(4-hydroxyphenyl)benzo[b]furan
5-Hydroxy-2-(4-hydroxyphenyl)-3-(6-N-piperidylhexyl)-benzo[b]furan
6-Hydroxy-2-(4-hydroxyphenyl)-3-(6-N-piperidylhexyl)-benzo[b]furan
6-Hydroxy-2-(4-hydroxyphenyl)-3-methylbenzo[b]thiophen
3-Ethyl-6-hydroxy-2-(4-hydroxyphenyl)benzo[b]thiophen
6-Hydroxy-2-(4-hydroxyphenyl)-3-propylbenzo[b]thiophen
6-Hydroxy-2-(4-hydroxyphenyl)-3-(6-N-piperidinylhexyl)-benzo[b]thiophen

Die Erfindung betrifft ferner ein Verfahren zur Herstellung der 2-Phenylbenzo-[b]furane und -thiophene der allgemeinen Formel I.

Dabei wird (werden)
a) wenn X letztendlich Sauerstoff sein soll eine Verbindung der allgemeinen Formel IIa 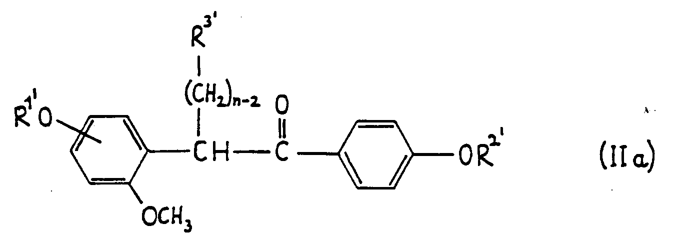 worin
   R^{1'} und R^{2'} unabhängig voneinander je eine Alkylgruppe mit 1 bis 4 Kohlenstoffatomen sowie
   R^{3'} entweder -(CH₂)₂-R, -(CH₂)₂-Hal, wobei Hal ein Halogenatom und insbesondere ein Bromatom ist, einen Vinylrest -CH=CH₂ oder einen Rest -CH₂-C(O)NR⁷R⁸
   bedeuten
   mit einer Lewis-Säure unter Spaltung der Ethergruppierungen zu einer Verbindung der allgemeinen Formel Ia 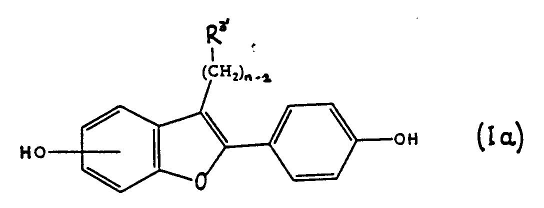 cyclisiert und wenn R^{3'}-(CH₂)₂-Hal bedeutet, Hal durch Umsetzung der Verbindung der Formel Ia mit einem primären, sekundären oder cyclischen Amin der Formel HNR⁷R⁸ gegen den entsprechenden Aminrest -NR⁷R⁸ ausgetauscht oder wenn R^{3'} einen Vinylrest bedeutet, durch endständige Hydroxylierung der vinylischen Doppelbindung, Überführung der erzeugten Hydroxygruppe in eine bessere Abgangsgruppe und diese analog zum Fall, daß R^{3'} ein Halogenatom bedeutet gegen einen Aminrest -NR⁷R⁸ ausgetauscht oder wenn R^{3'} den Rest -CH2-C(O)NR7R8 bedeutet, die Carbonylgruppe mit Lithiumaluminiumhydrid vollständig reduziert und die freien Hydroxygruppen gegebenenfalls verethert oder verestert, oder
b) wenn X letztendlich Schwefel sein soll, eine Verbindung der allgemeinen Formel IIb 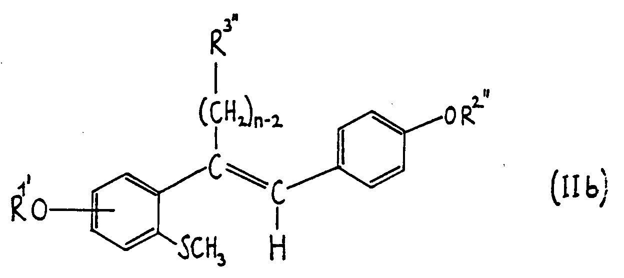 worin
   R^{1'} und R^{2'} dieselbe Bedeutung wie in Formel IIa haben und R^{3''}-(CH₂)₂-R, -(CH₂)₂-Hal ein Halogenatom und insbesondere ein Bromatom ist, oder einen Vinylrest -CH=CH₂
   bedeutet,
   mit einem Gemisch aus Sulfurylchlorid und Pyridin zu dem entsprechenden Benzo[b]thiophenderivat der allgemeinen Formel Ib 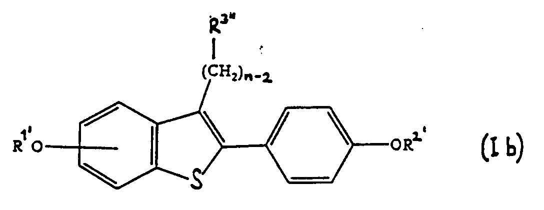 cyclisiert und dann wenn R^{3''}-(CH₂)₂-Hal oder einen Vinylrest -CH=CH₂ bedeutet wie unter a) für diese beiden Fälle angegeben die Verbindung der allgemeinen Formel I b weiter umgesetzt und anschließend mit einer Lewis-Säure die Ethergruppierungen gegebenenfalls gespalten und die freien Hydroxygruppen gegebenenfalls verethert oder verestert,
c) wenn X letztendlich Schwefel sein soll, eine Verbindung der allgemeinen Formel II c 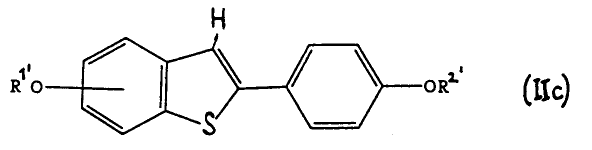 worin R^{1'} und R^{2'} dieselbe Bedeutung wie in Formel II a haben,
   mit einem Säurehalogenid der allgemeinen Formel 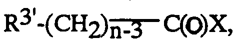 wobei R^{3'} und n dieselbe Bedeutung wie in Formel II a haben und X ein Chlor- oder Bromatom ist, acyliert und nachfolgend mit LiAlH₄/AlCl₃ zu einer Verbindung der allgemeinen Formel I c 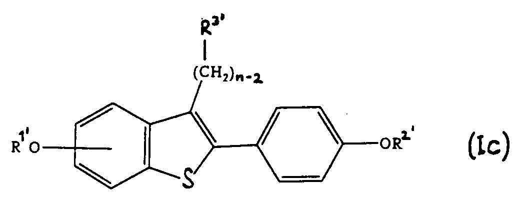 reduziert und wie unter a) beschrieben weiter umgesetzt.

Für die Alkylgruppen R¹'und R²' mit 1 bis 4 Kohlenstoffatomen kommen die Ethyl-, Propyl-Isopropyl-, Butyl-, Isobutyl-, tert.-Butyl- und insbesondere die Methylgruppe infrage.
Die Cyclisierung der Verbindungen der allgemeinen Formel IIa zu Verbindungen der allgemeinen Formel Ia mit einer Lewis-Säure verläuft unter (gleichzeitiger) Spaltung der Ethergruppierungen R¹'-O- und R²'-O- und Bildung der entsprechenden freien Hydroxyverbindungen.

Als Reagenz für die Cyclisierung und Etherspaltung eignen sich Bortribromid, Bortrifluorid, Aluminiumtrichlorid, Siliziumtetrachlorid, Aluminiumtribromid, Natriummethylthiolat und Trimethylsilyljodid. Die Reaktion wird bei Temperaturen zwischen -70°C und 200°C durchgeführt. Als Lösungsmittel für diese Cyclisierung und Etherspaltung kommen inerte Lösungsmittel infrage. Zu nennen sind aliphatische Halogenkohlenwasserstoffe, wie zum Beispiel Methylenchlorid, aromatische Kohlenwasserstoffe, wie Chlorbenzol, Dichlorbenzole und Dimethylformamid sowie Acetonitril. Es eignen sich aber auch aliphatische Ether mit Alkylresten aus 1-6 Kohlenstoffatomen.

Für die gegebenenfalls durchzuführende Veresterung der phenolischen Hydroxylgruppen kommen die üblicherweise in der Chemie zur Veresterung angewendeten Verfahren infrage. Beispielsweise sei die Umsetzung mit einer Carbonsäure oder eines Carbonsäureanhydrides in Gegenwart starker Säuren, wie zum Beispiel Trifluoressigsäure, Perchlorsäure oder p-Toluolsulfonsäure, bei Raumtemperatur oder etwas angehobener Temperatur oder die Umsetzung mit einem Carbonsäureanhydrid in Gegenwart eines tertiären Amins bei etwa 20-80°C zu nennen.

Werden Pyridin und 4-Dimethylamino-pyridin als tertiäre Amine gemeinsam angewandt, kann die Veresterung vorzugsweise bei Raumtemperatur durchgeführt werden. Die übrigen Reaktionsschritte zur Herstellung der erfindungsgemäßen Benzo[b]furane der allgemeinen Formel I (X=0) werden nach Standardmethoden der organischen Chemie durchgeführt. Die Kopplung des basischen Fragments -NR⁷R⁸
an die Verbindungen der allgemeinen Formel Ia, worin R3'-(CH2)2-Hal bedeutet, erfolgt durch Kochen der ω-Halogenverbindung in dem entsprechenden Amin.

Die selektive endständige Hydroxylierung der vinylischen Doppelbindung, d.h. also wenn R^{3'}-CH=CH₂ ist, wird durch Hydroborierung, beispielsweise mit 9-Borbicyclo[3.3.1]nonan erreicht. Die gebildete ω-Hydroxyverbindung wird dann beispielsweise durch Behandlung mit Methansulfonylchlorid und Triethylamin in das entsprechende Mesylat überführt. Die Hydroxygruppe kann selbstverständlich auch gegen andere, gute Abgangsgruppen, beispielsweise gegen ein Bromatom, den Tosylrest, die Trifluormethansulfonatgruppe oder ähnliche Abgangsgruppen ausgetauscht werden. Die Ankopplung des basischen Fragments erfolgt wie oben beschrieben, wobei die Temperatur je nach Reaktivität der Abgangsgrupe anzupassen ist.

Zur Herstellung der Benzo[b]thiophen der allgemeinen Formel I (X = S) wird ein Orthothiomethylstyrol der allgemeinen Formel IIb mit einem Gemisch aus Sulfurylchlorid und Pyridin cyclisiert. Dabei reagiert das Orthothiomethylstyrol zum entsprechenden Sulfoniumchlorid, das mit Pyridin unter Abspaltung von Methylchlorid in das entsprechende Chlorsulfid übergeht und anschließend unter HCl-Verlust zum entsprechenden Benzo[b]thiophen cyclisiert. Die sich gegebenenfalls anschließende weitere Umsetzung wird wie bei den Benzo[b]furanen beschrieben durchgeführt.

Alternativ kann vom in 3-Stellung unsubstituierten Heterocyclus ausgegangen werden und die Seitenkette durch Friedl-Crafts-Acylierung und nachfolgender Reduktion mit LiAlH₄/AlCl₃ eingeführt werden.

Es wurde gefunden, daß die erfindungsgemäßen Verbindungen der allgemeinen Formel I starke antiestrogene Eigenschaften besitzen.
Verbindungen mit antiestrogenen Eigenschaften, d.h. Stoffe mit Hemmwirkungen gegenüber Estrogenen, werden bereits in der Literatur beschrieben.
Als Antiestrogen ist beispielsweise das Tamoxifen zu nennen (Eur. J. Cancer Clin. Oncol, 1985, 21, 985 und J.S. Patterson, "10 Years of Tamoxifen in Breast Cancer" in Hormonal Manipulation of Cancer; Peptides, Growth Factors and New (Anti) steroidal Agents, Raven Press, New York (1987)).

Steroidale Antiestrogene werden in der europäischen Patentanmeldung 0 138 504 beschrieben. Antiestrogene Indolderivate gehen bereits aus der deutschen Patentschrift 32 32 968, aus J. Med. Chem. 1983, 26, 113; J. Med. Chem., 1984, 27, 1439, Eur. J. Cancer. Clin. Oncol. 1985, 21, 531 und Cancer Treatment Reviews 1984, 11, 147 sowie N-Aminoalkylindole, die neben ausgeprägter antiestrogener Wirksamkeit nur noch geringe estrogene Aktivität aufweisen, aus der europäischen Patentanmeldung 0 348 341 hervor.

Hydroxylierte 2-Phenylindole, die in Form von Diamin-platin (II)-Komplex-Verbindungen vorliegen, werden in der deutschen Offenlegungsschrift 37 30 746 genannt.

3-unsubstituierte Benzo[b]thiophene sind aus EP-A-0 062 503, EP-A-0 062 504, EP-A-0 062 505 und aus Journal of Medicinal Chemistry, Bd. 27, 1984, Seiten 1057 - 1066 bekannt.

3-unsubstituierte und 3-Methyl-substituierte Benzo[b]furane sind in Chimica Therapeutica, Bd. 4, 1973, Seiten 398 - 411 und in Journal of Medicinal Chemistry, Bd. 32, 1989, Seiten 1700 - 1707 sowie ausschließlich 3-Methyl-substituierte Benzo[b]furane in JP-A-0 248 524 (entsp. Chem. Abstr., 113, No. 5, ref.: 34694w) beschrieben.

Die erfindungsgemäßen Verbindungen der allgemeinen Formeln I besitzen eine ausgeprägte Affinität zum Estradiol-Rezeptor und verdrängen kompetiv ³H-17β-Estradiol vom Rezeptor. In vivo besitzen sie starke antiestrogene Effekte am Uterus der Maus und hemmen das estrogen-stimulierende Uteruswachstum bis zu 100%. Estrogene Wirkungen sind in diesen Tests nicht oder nur in sehr geringem Maße nachweisbar. Die Verbindungen wirken hemmend auf das Wachstum von hormonabhängigen Tumorzellen, insbesondere hemmen sie das Wachstum von estrogen-abhängigen menschlichen Mammatumorzellen (MCF-7).

Die erfindungsgemäßen Verbindungen eignen sich zur Therapie von estrogen-abhängigen Erkrankungen, zum Beispiel Prostatahyperplasie, Mammacarcinom, Endometriumcarcinom, anovulatorische Infertilität und Melanom.

Die folgenden pharmakologischen Tests zeigen die Wirksamkeit der erfindungsgemäßen Verbindungen.

Tabelle 1 zeigt eine Übersicht der getesteten Verbindungen der allgemeinen Formel I und deren relativen Bindungsaffinitäten (RBA*) zum Estrogenrezeptor aus Kalbsuteri, bezogen auf 17β-Estradiol = 100.

Die Testanordnung wird in Cancer Treatment Reviews 1984, 11, 147 beschrieben.

Aus der Tabelle 1 geht hervor, daß die Verbindungen 104a, 104b, 102a, 106b, 106a, 63a und 62a die größten Affinitäten im Vergleich zu Estradiol zeigen

Tabelle 2 zeigt die estrogenen und antiestrogenen Wirksamkeiten der Verbindungen 62a, 62b, 68a, 68b, 102a, 102b, 104a, 104b, 106a und 106b. Diese Wirksamkeiten wurden in einem in-vivo-Test an der infantilen Maus gefunden. Dieser Test wird ausführlich in Cancer Treatment Reviews 1984, 11, 147 und J. Med. Chem., 1984, 27, 1439 beschrieben.

Tabelle 3 zeigt die Ergebnisse der Untersuchungen zur cytostatischen Aktivität der Verbindungen 61a, 62a, 63a, 68a und 68b sowie von 102a, 104a, 106a und 102b, 104b, 106b, 119b im Vergleich zu Tamoxifen.
An hormonsensitiven menschlichen MCF-7 Mammacarcinomzellen wurde eine starke Hemmung des Zellwachstums gefunden.

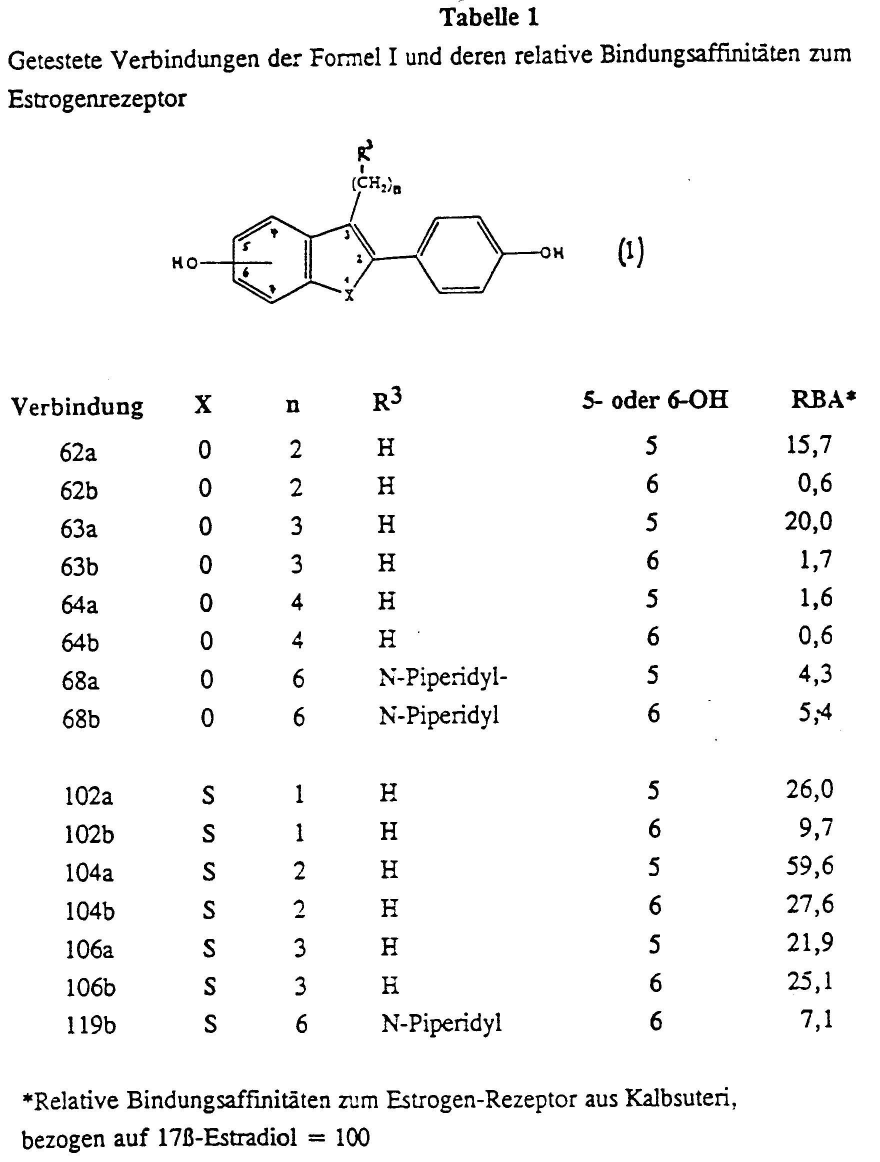

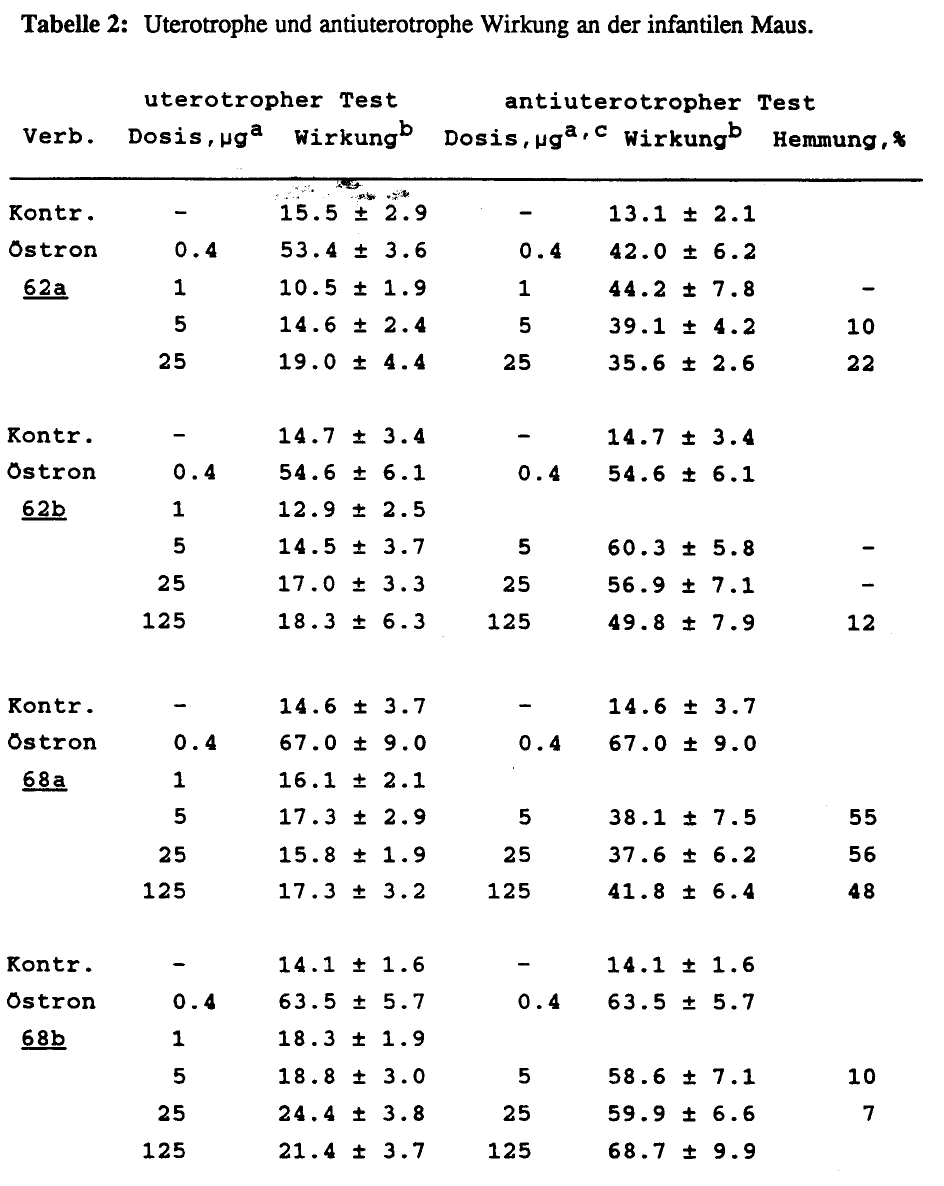

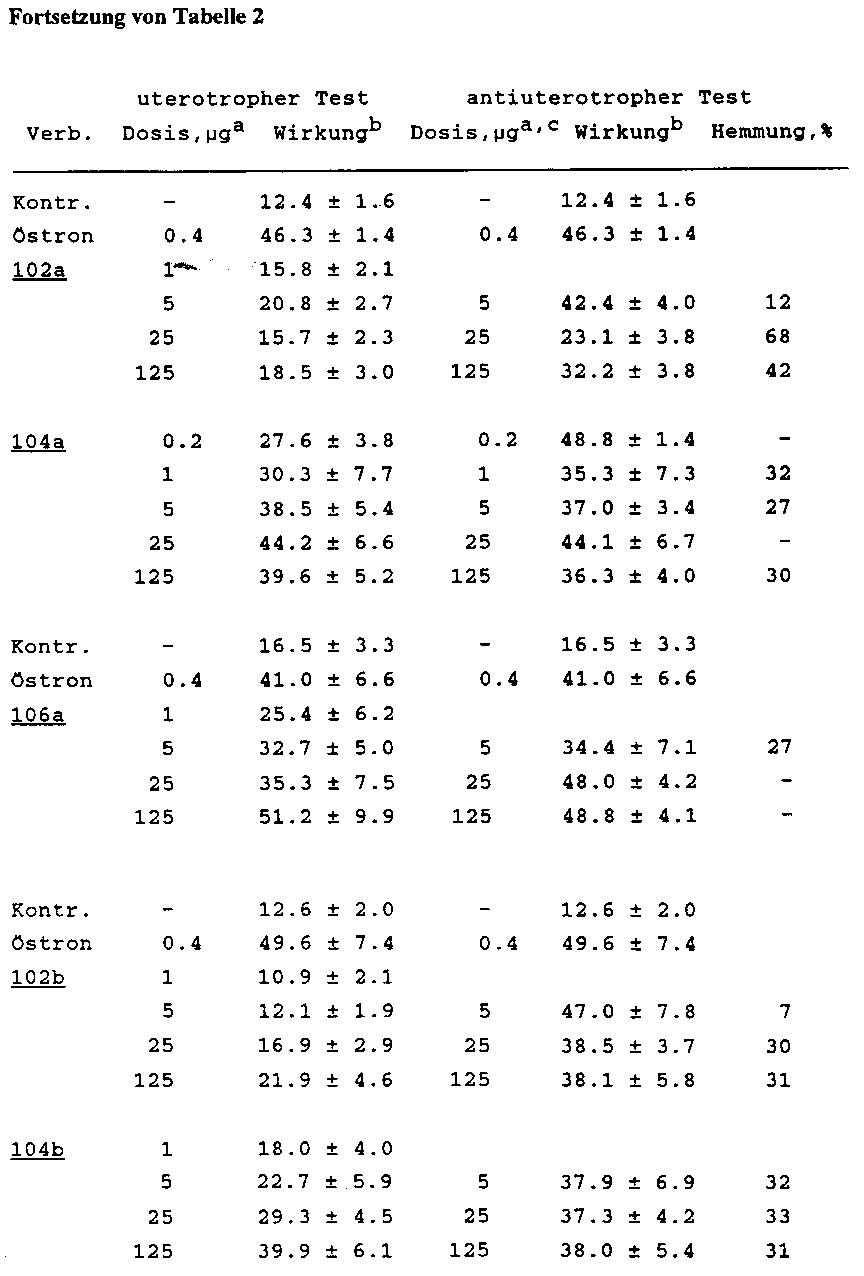

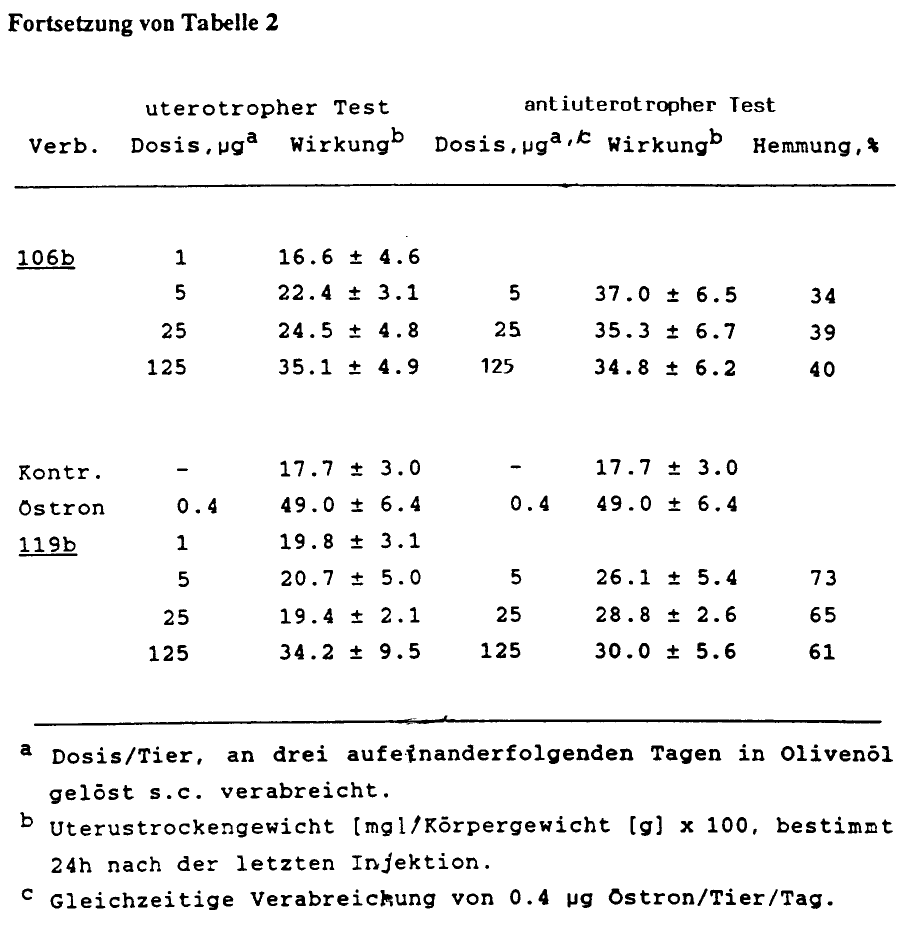

**Tab. 3**

| Wirkung von 2-Phenylbenzo[b]furanen und -Thiophenen auf das Zellwachstum der MCF 7 Zellinie. Daten als korrigierte T/C-Werte (%) angegeben | | | | |
|---|---|---|---|---|
| Verb. | T/C^{a} [%] | | | |
| | 1 x 10⁻⁷ M^{b} | 1 x 10⁻⁶ M^{b} | 5 x 10⁻⁶ M^{b} | 1 x 10⁻⁵ M^{b} |
| Tam | 56.5 ± 16.3^{c} | 59.1 ± 14.1^{c} | 33.6 ± 10.3^{c} | -5.7 ± 3.4^{c} |
| 62a | 65.0 ± 13.5^{c} | 49.0 ± 12.6^{c} | 0.3 ± 3.8^{c} | -0.2 ± 2.9^{c} |
| 63a | 51.0 ± 11.3^{c} | 50.5 ± 8.8^{c} | -0.1 ± 3.6^{c} | -2.1 ± 2.8^{c} |
| 68a | 64.4 ± 12.0^{c} | 51.6 ± 10.2^{c} | 10.4 ± 4.8^{c} | -9.9 ± 1.8^{c} |
| 68b | 58.7 ± 17.1^{c} | 42.9 ± 7.7^{c} | 12.1 ± 5.4^{c} | -9.6 ± 2.9^{c} |
| Tam | 56.5 ± 16.3^{c} | 59.1 ± 14.1^{c} | 33.6 ± 10.3^{c} | -5.7 ± 3.4^{c} |
| 102a | 71.3 ± 18.8 | 87.4 ± 20.0 | 48.5 ± 14.7^{c} | 14.7 ± 6.4^{c} |
| 104a | 61.2 ± 22.1^{c} | 59.1 ± 15.9^{c} | 2.4 ± 3.9^{c} | 3.2 ± 3.2^{c} |
| 106a | 77.1 ± 21.4 | 35.0 ± 10.4^{c} | 2.3 ± 4.1^{c} | 0.3 ± 3.5^{c} |
| 102b | 124.6 ± 37.2 | 133.3 ± 43.4 | 54.9 ± 17.9^{c} | 10.9 ± 6.7^{c} |
| 104b | 94.0 ± 21.2 | 91.9 ± 21.2 | 5.7 ± 5.8^{c} | 4.2 ± 6.0^{c} |
| 106b | 81.7 ± 17.5 | 69.0 ± 15.1^{c} | 3.6 ± 3.0^{c} | 1.8 ± 3.1^{c} |
| 119b | 18.8 ± 7.2^{c} | 48.1 ± 12.4^{c} | 2.0 ± 3.6^{c} | -13.5 ± 3.2^{c} |

| | | | | |
|---|---|---|---|---|
| ^{a} Hemmwirkung an MCF-7 Zellen; Quotient der optischen Dichten aus Test-(T) und Kontrollgruppe (C); Mittelwert aus 16 Einzelbestimmungen; Standardabweichung durch Fehlerberechnung nach GAUSS. | | | | |
| ^{b} Substanzkonzentration [mol/l] im Inkubationsmedium. | | | | |
| ^{c} Signifikant p < 0.01 gegenüber der Kontrollgruppe (C). | | | | |

Die Erfindung betrifft auch pharmazeutische Präparate, die mindestens eine Verbindung der allgemeinen Formel I enthalten und
die Verwendung dieser Verbindungen zur Behandlung von östrogenabhängigen Krankheiten und Tumoren.

Die erfindungsgemäßen Verbindungen sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arneimittel erfolgt in bekannter weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 u.ff.,
H.v.Czetsch-Lindenwald, Hilfsstoffe für Pharmazie und angrenzende Gebiete; Pharm. Ind., Heft 2, 1961, Seite 72 u.ff.; Dr. H.P. Fiedler, Lexikon der Hilfsstoffe für Pharmazie, Kosmetik und angrenzende Gebiete Cantor KG. Aulendorf in Württemberg 1971.

Die Verbindungen können oral oder parenteral, beispielsweise intraperitoneal, intramuskulär, subkutan oder perkutan, verabreicht werden. Die Verbindungen können auch in das Gewebe implantiert werden. Die zur verabreichende Menge der Verbindung schwankt innerhalb eines weiten Bereichs und kann jede wirksame Menge abdecken. In Abhängigkeit des zu behandelnden Zustands und der Art der Verabreichung kann die Menge der verabreichten Verbindung 0,01-100 mg/kg Körpergewicht, vorzugsweise 0,1-20 mg/kg Körpergewicht, je Tag betragen.

Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten. Die einzelnen Dosierungseinheiten für die orale Applikation können beispielsweise 10 bis 100 mg des Wirkstoffs enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl. Erdnußöl, Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Die nachfolgenden Arbeitsvorschriften und Beispiele dienen der näheren Erläuterung der Erfindung.
Die Reaktionsschemata zur Herstellung der erfindungsgemäßen Verbindungen sowie der hierfür benötigten Zwischenprodukte sind den Abb. 1 bis 4 zu entnehmen.

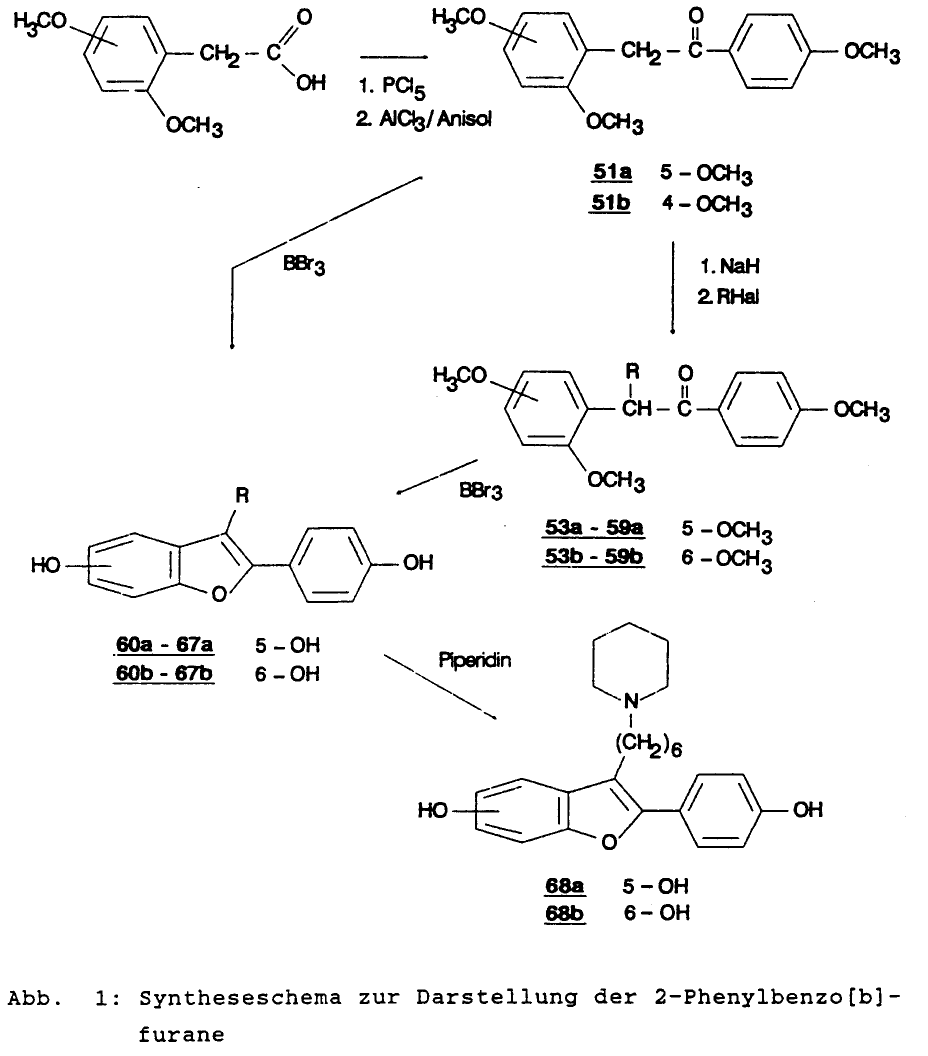

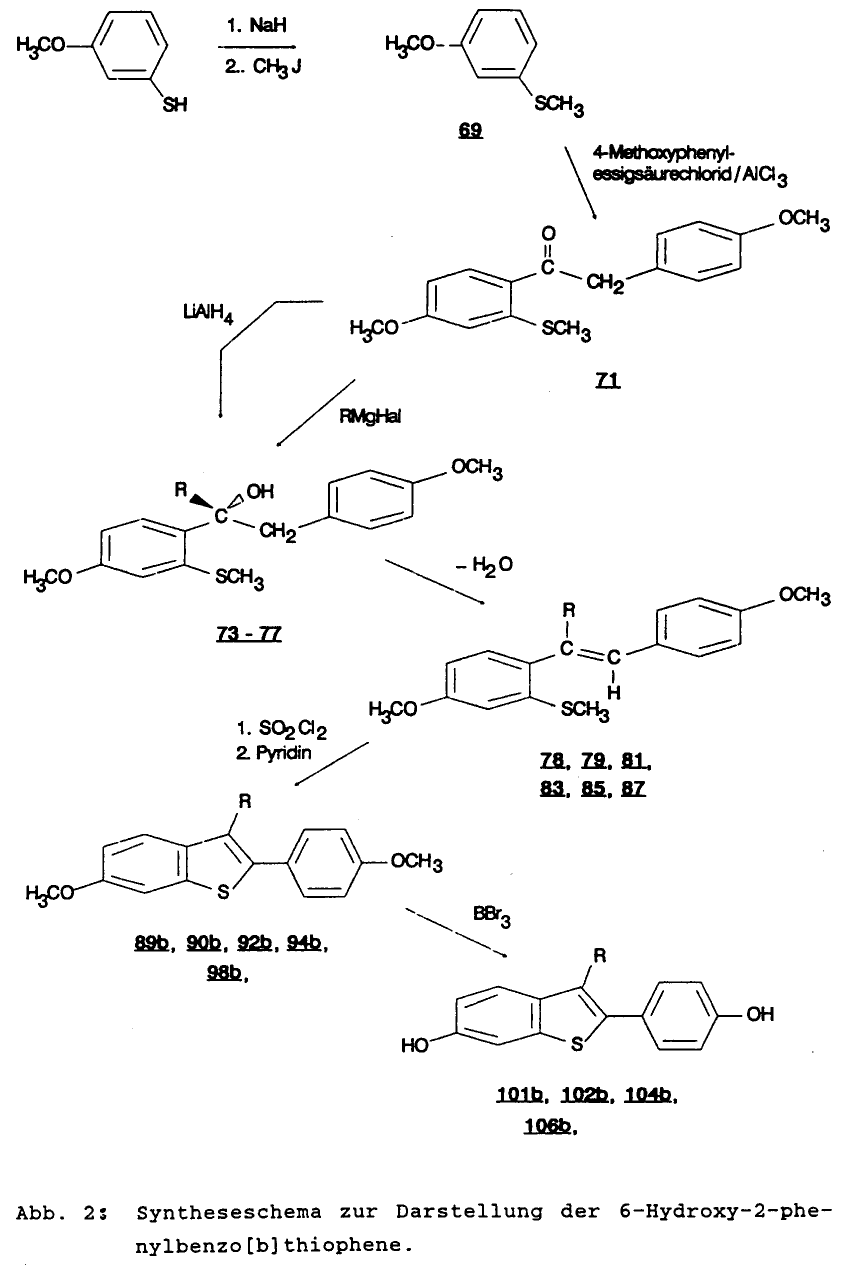

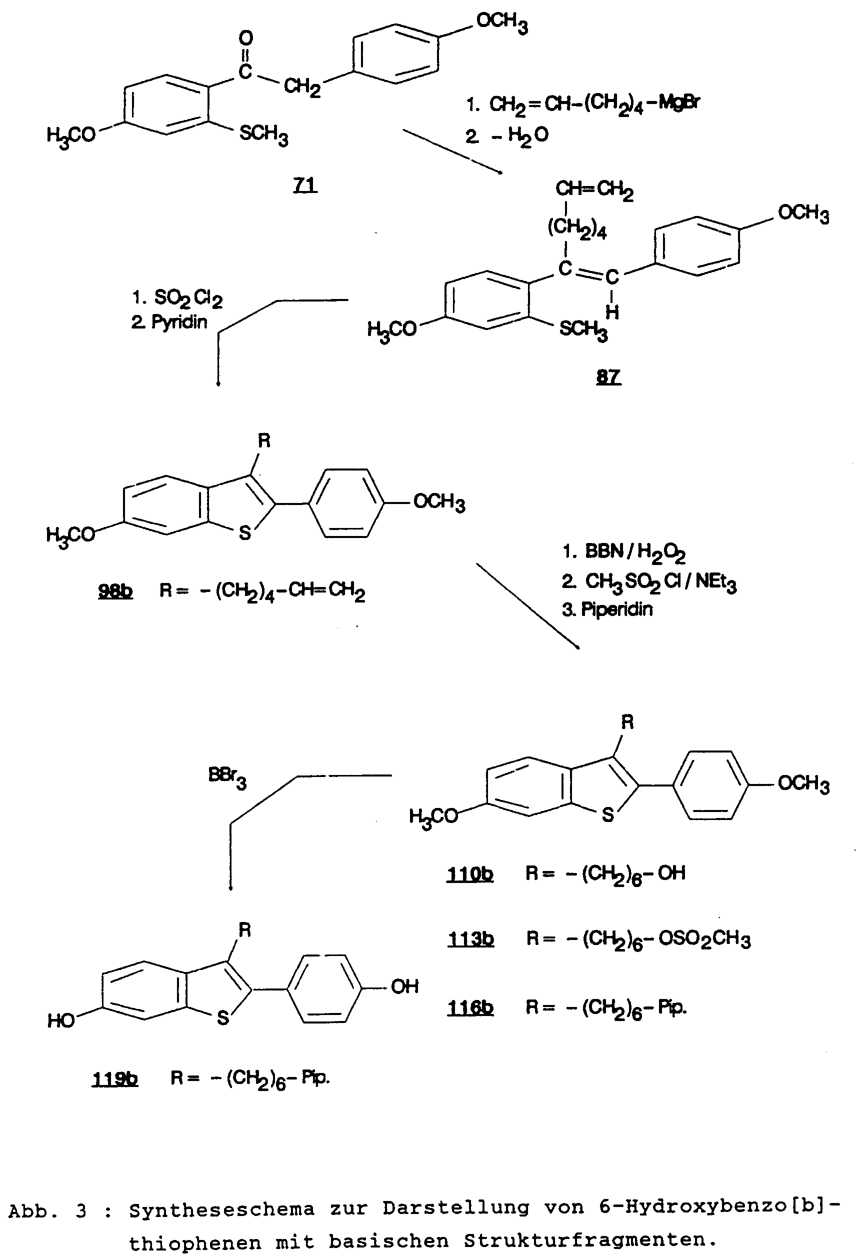

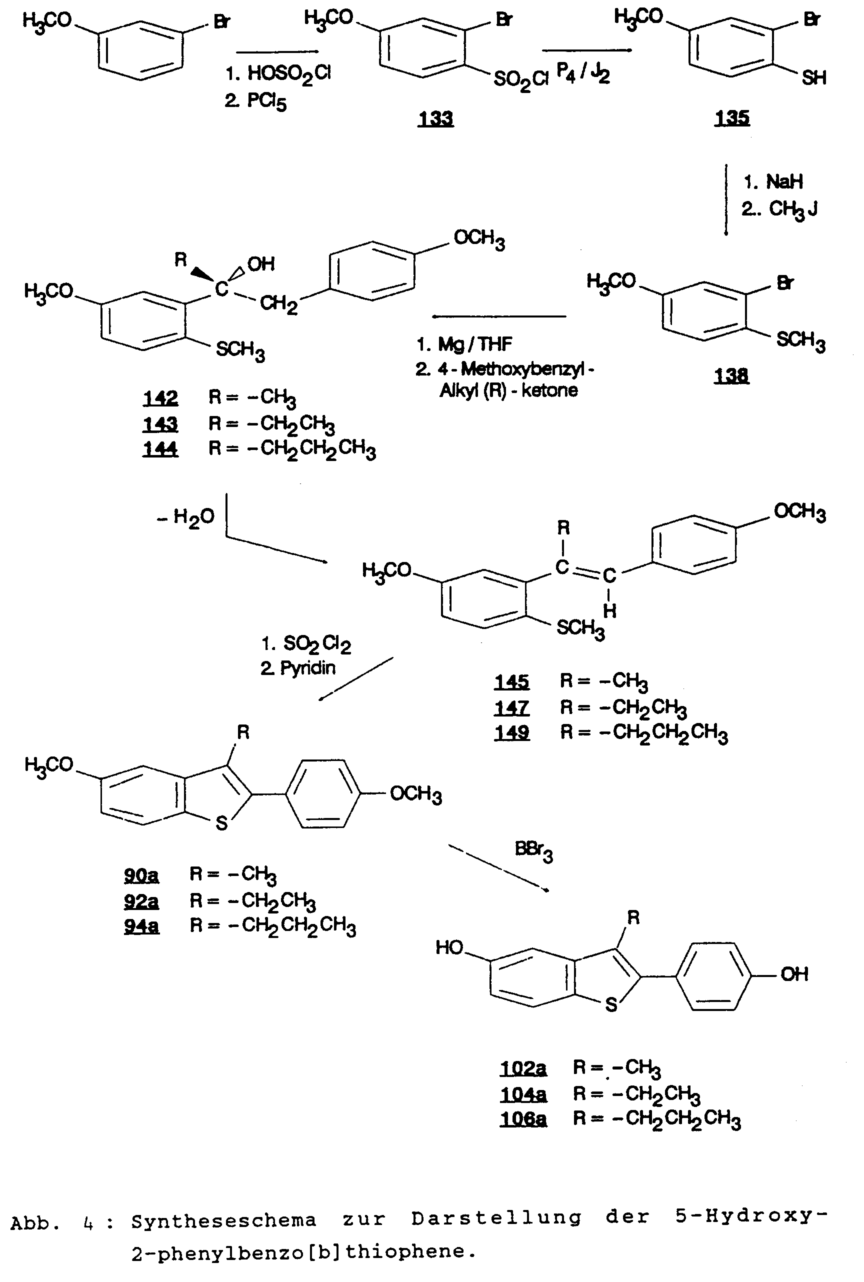

### 2,4-Dimethoxyphenylessigsäure (48)

In einem 250 ml Rundkolben mit Rückflußkühler werden 20.0 g (0.11 mol) 2,4-Dimethoxyacetophenon, 19.32 g (0.22 mol) Morpholin (19.3 ml) und 7.12 g (0.22 mol) Schwefel ca 20 Stunden auf 135° C erhitzt. Anschließend wird überschüssiges Morpholin im Vakuum entfernt. Das zurückbleibende Thiomorpholid wird ohne weitere Reinigung verseift.
Verseifung: Das braune Öl wird mit 90.0 g 50%iger KOH in 160.0 ml Ethanol versetzt und 6 Stunden Rückfluß erhitzt. Anschließend destilliert man den Alkohol weitgehend ab, verdünnt mit Wasser und filtriert feste Bestandteile ab. Unter Eiskühlung wird mit konzentrierter Salzsäure angesäuert, dreimal mit Dichlormethan extrahiert, über MgSO4 getrocknet, filtriert und im Wasserstrahlvakuum eingeengt. Das Rohprodukt wird aus Wasser umkristallisiert.
beige Kristalle; Schm.: 106-108°C; Ausbeute: 51%

### Darstellung der Säurechloride

Ein Gemenge aus 0.5 mol Carbonsäure und 0.5 mol Phosphorpentachlorid wird unter Eiskühlung 1/2 Stunde gerührt. Anschließend erwärmt man 1 Stunde auf 60° und zieht das entstandene Phosphorylchlorid im Vakuum ab. Der Rückstand wird mit absolutem Benzol versetzt und erneut eingeengt, um restliches Phosphorylchlorid zu entfernen. Der Rückstand wird ohne weitere Reinigung umgesetzt.

### 2,5-Dimethoxyphenylessigsäurechlorid (50a)

farbloses Öl; Ausbeute: 97%
IR (Film): 1805 cm⁻¹ (s; C = O)

### 2,4-Dimethoxyphenylessigsäurechlorid (50b)

gelbes Öl; Ausbeute 95%
IR (Film): 1810 cm⁻¹ (s; C =O)

### Friedel-Crafts-Acylierung

Eine Lösung von 0.05 mol Säurechlorid in 150 ml 1,2-Dichlorethan wird mit 10,8 g (0.1 mol) Anisol versetzt. Unter Rühren und Eiskühlung gibt man portionsweise 13.3 g (0.1 mol) Aliminiumtrichlorid zu. Man läßt über Nacht bei Raumemperatur rühren und gießt dann auf ca. 200 ml Eiswasser. Nach dem Abtrennen der organischen Phase wird die wäßrige Phase dreimal mit Dichlormethan extrahiert. Die vereinigten organischen Phasen werden zweimal mit 10%iger Natronlauge und dreimal mit Wasser gewaschen, über MgSO4 getrocknet, filtriert und im Vakuum eingeengt. Der Rückstand wird mittels Säulenchromatographie (Kieselgel 60; Dichlormethan) gereinigt und aus Ethanol umkristallisiert.

### 2-(2,5-Dimethoxyphenyl)-1-(4-methoxyphenyl)ethanon (51a)

Edukte: 2,5-Dimethoxyphenylessigsäurechlorid (50a) Anisol
farblose Kristalle; Schmp.: 105-107°C Ausbeute: 76%

### 2-(2,4-Dimethoxyphenyl)-1-(4-methoxyphenyl)ethanon (51b)

Edukte: 2,4-Dimethoxyphenylessigsäurechlorid (50b) Anisol
farblose Kristalle; Schmp.: 100-102°C Ausbeute: 48%

### Darstellung der 2-Alkyl-1,2-diarylethanone

Unter Eiskühlung werden 0.5 g (21.0 mol) Natriumhydrid (80% in Paraffin) in 80 ml absolutem Dimethylformamid (DMF) suspendiert und 15 min. bei 0°C gerührt. Anschließend wird eine Lösung von 4.0 g (14.0 mmol) 1,2-Diarylethanon in 40 ml absolutem DMF zugetropft und bis zum Abklingen der Gasentwicklung gerührt (ca. 30 min.). Der gekühlten Mischung wird eine Lösung von 21.0 mmol Alkylhalogenid in 20 ml absolutem DMF zugetropft. Man rührt 1/2 Stunde bei 0°C, entfernt das Kältebad und rührt 1/2 Stunde bei Raumtemperatur. Überschüssiges Natriumhydrid wird durch Eingießen in Eiswasser vernichtet. Das Hydrolysat wird dreimal mit Ether extrahiert, zweimal mit Wasser gewaschen, über MgSO4 getrocknet, filtriert und im Wasserstrahlvakuum eingeengt. Der Rückstand wird mit Dichlormethan über Kieselgel 60 chromatographiert. Kristalline Produkte wurden aus Ethanol umkristallisiert.

### 2-(2,5-Dimethoxyphenyl)-1-(4-methoxyphenyl)propanon (53a)

Edukte: 2-(2,5-Dimethoxyphenyl)-1-(4-methoxyphenyl)ethanon (51a) Methyljodid
farblose Kristalle; Schmp.: 97-98°C Ausbeute: 71%

### 2-(2,4-Dimethoxyphenyl)-1-(4-methoxyphenyl)propanon (53b)

Edukte: 2-(2,4-Dimethoxyphenyl)-1-(4-methoxyphenyl)ethanon (51b) Methyljodid
farblose Kristalle; Schmp.: 59-60°C Ausbeute: 38%

### 2-(2,5-Dimethoxyphenyl)-1-(4-methoxyphenyl)butanon (54a)

Edukte: 2-(2,5-Dimethoxyphenyl)-1-(4-methoxyphenyl)ethanon (51a) Ethyljodid
gelbes Öl; Ausbeute: 67%
IR (Film): 1680 cm⁻¹ (s; C = O)

### 2-(2,4-Dimethoxyphenyl)-1-(4-methoxyphenyl)butanon (54b)

Edukte: 2-(2,4-Dimethoxyphenyl)-1-(4-methoxyphenyl)ethanon (51b) Ethyljodid
gelbes Öl; Ausbeute: 69%
IR (Film) 1680 cm⁻¹ (s; C = O)

### 2-(2,5-Dimethoxyphenyl)-1-(4-methoxyphenyl)pentanon (55a)

Edukte: 2-(2,5-Dimethoxyphenyl)-1-(4-methoxyphenyl)ethanon (51a) Propyljodid
gelbes Öl; Ausbeute: 81%
IR (Film) 1680 cm⁻¹ (s; C = O)

### 2-(2,4-Dimethoxyphenyl)-1-(4-methoxyphenyl)pentanon (55b)

Edukte: 2-(2,4-Dimethoxyphenyl)-1-(4-methoxyphenyl)ethanon (51b) Propyljodid
gelbes Öl; Ausbeute: 72%
IR (Film) 1680 cm⁻¹ (s; C = O)

### 2-(2,5-Dimethoxyphenyl)-1-(4-methoxyphenyl)hexanon (56a)

Edukte: 2-(2,5-Dimethoxyphenyl)-1-(4-methoxyphenyl)ethanon (51a) Butyljodid
farblose Kristalle; Schmp.: 62-63°C Ausbeute: 83%

### 2-(2,4-Dimethoxyphenyl)-1-(4-methoxyphenyl)hexanon (56b)

Edukte: 2-(2,4-Dimethoxyphenyl)-1-(4-methoxyphenyl)ethanon (51b) Butyljodid
gelbes Öl; Ausbeute: 77%
IR (Film) 1680 cm⁻¹ (s; C = O)

### 8-Brom-2-(2,5-dimethoxyphenyl)-1-(4-methoxyphenyl)octanon (59a)

In diesem Fall wird die gekühlte Mischung aus 1,2-Diarylethanon und Natriumhydrid zur 1,6-Dibromhexanlösung getropft. Als Edukte dienten 2-(2,5-Dimethoxyphenyl)-1-(4-methoxyphenyl)ethanon (51a) und 1,6-Dibromhexan.
farbloses Öl; Ausbeute: 61%
IR (Film) 1675 cm⁻¹ (s; C = O)

### 8-Brom-2-(2,4-dimethoxyphenyl)-1-(4-methoxyphenyl)octanon (59b)

In diesem Fall wird die gekühlte Mischung aus 1,2-Diarylethanon und Natriumhydrid zur 1,6-Dibromhexanlösung getropft. Als Edukte dienten 2-(2,4-Dimethoxyphenyl)-1-(4-methoxyphenyl)ethanon (51b) und 1,6-Dibromhexan.
gelbliches Öl; Ausbeute: 48%
IR (Film) 1675 cm⁻¹ (s; C = O)

### Methylierung von Thiophenolen

In 100 ml absolutem Dimethylformamid (DMF) werden 5.3 g (0.22 mol) Natriumhydrid suspendiert und 30 Minuten im Eisbad gerührt. Bei 0°C werden langsam 0.15 mol des Thiophenols in 50 ml absolutem DMF zugetropft. Die Mischung wird gerührt bis keine merkliche Gasentwicklung erkennbar ist. Anschließend wird unter Eiskühlung eine Lösung von 22.7 g (0.16 mol; 2.44 ml) Methyljodid in 50 ml absolutem DMF zugetropft. Man rührt 30 Minuten bei 0°C, erwärmt auf Raumtemperatur und läßt weitere 60 Minuten rühren. Überschüssiges Natriumhydrid wird durch Eingießen in Eiswasser vernichtet. Das Hydrolysat wird dreimal mit Ether extrahiert. Die vereinigten organischen Phasen werden gründlich mit Wasser gewaschen, über MgSO4 getrocknet, filtriert und im Vakuum eingeengt. Der Rückstand wird mittels Säulenchromatographie (Kieselgel 60; Dichlormethan) oder durch Destillation im Vakuum gereinigt.

### 3-Methoxyphenyl-methyl-sulfid (69)

Edukte: 3-Mercaptoanisol; Methyljodid
farbloses Öl; Sdp.: 57-58°C (0.1 mm) Ausbeute: 95%
¹H-NMR(CDCl₃): d = 2.47 (s;3H, -SCH₃); 3.78 (s;3H, -OCH₃); 6.58-6.97 (m;3H, ArH); 7.22 (t;³J = 8 Hz; 1H, ArH).

### 4-Methoxyphenylessigsäurechlorid (70)

Darstellung erfolgt gemäß vorstehender Vorschrift "Darstellung der Säurechloride"
farbloses Öl; Sdp.: 79-81°C (0.1 mm); Ausbeute: 96%
IR (Film): 1800 cm⁻¹ (s; C = O)

### 1-(4-Methoxy-2-methylthiophenyl)-2-(4-methoxyphenyl)ethanon (71)

Die Synthese erfolgt analog der vorstehenden allgemeinen Arbeitsvorschrift für die Friedel-Crafts-Acylierung.
Edukte: 3-Methoxyphenyl-methyl-sulfid (69) 4-Methoxyphenylessigsäurechlorid (70)
SC: Kieselgel 60; Dichlormethan
farblose Kristalle (EtOH); Schmp. 88-89°C; Ausbeute: 48%

### 1-(4-Methoxy-2-methylthiophenyl)-2-(4-methoxyphenyl)ethanol (72)

In 50.0 ml absolutem Ether werden 0.3 g (7.9 mmol) Lithiumaluminiumhydrid vorgelegt und im Eisbad auf 0-5°C gekühlt. 1-(4-Methoxy-2-methylthiophenyl)-2-(4-methoxyphenyl)ethanon (71) (6,8 mml) werden in absolutem Ether gelöst und langsam zur Lithiumaluminiumhydridsuspension zugetropft. Anschließend wird 1 h zum Sieden erhitzt. Nach dem Erkalten wird vorsichtig mit Wasser hydrolysiert und mit verdünnter Salzsäure angesäuert bis sämtliches Aluminiumhydroxyd in Lösung gegangen ist. Es wird dreimal mit Ether extrahiert, zweimal mit Wasser gewaschen, über MgSO₄ getrocknet, filtriert und im Wasserstrahlvakuum eingeengt. Das Produkt wird mittels Säulenchromatographie an
Kieselgel 60 gereinigt (Elutionsmittel Dichlormethan/Ether (19:1).
farblose Nadeln; Schmp. 69-70°C; Ausbeute: 93%

### Darstellung der 1-Alkyl-1,2-diarylethanole

Unter Stickstoffspülung werden 1,2 g (49.5 mmol) Magnesiumspäne mit geringen Mengen Jod durch Erwärmen aktiviert. Anschließend werden unter Stickstoff 49.5 mmol Alkylhalogenid in 20 ml absolutem Ether zu den aktivierten Magnesiumspänen getropft. Die Reaktion springt unter Aufsieden des Ethers an. Nach beendeter Alkylhalogenidzugabe wird die Mischung 1 Stunde refuxiert. Nach dem Erkalten setzt man langsam mit 5.0 g (16.5 mmol) 1-(4-Methoxy-2-methylthiophenyl)-2-(4-methoxyphenyl)ethanon (71) in 40 ml absolutem Ether und erhitzt 2 Stunden unter Rückfluß. Das erkaltete Reaktionsgemisch wird mit Wasser hydrolysiert, mit verdünnter Salzsäure angesäuert und dreimal mit Ether extrahiert. Die vereinigten organischen Phasen werden mit Wasser gewaschen, filtriert und im Wasserstrahlvakuum eingeengt. Der Rückstand wird mit Dichlormethan über Kieselgel 60 chromatographiert.

### 2-(4-Methoxy-2-methylthiophenyl)-1-(4-methoxyphenyl)propan-2-ol (73)

Edukte: 1-(4-Methoxy-2-methylthiophenyl)-2-(4-methoxyphenyl)ethanon (71) Methyljodid
gelbes Öl; Ausbeute: 82%
IR (Film): 3420 cm⁻¹ (m; br; -OH)

### 2-(4-Methoxy-2-methylthiophenyl)-1-(4-methoxyphenyl)-butan-2-ol (74)

Edukte: 1-(4-Methoxy-2-methylthiophenyl)-2-(4-methoxyphenyl)ethanon (71) Ethyljodid
gelbes öl; Ausbeute: 78%
IR (Film): 3560 cm⁻¹ (m; br; -OH)

### 2-(4-Methoxy-2-methylthiophenyl)-1-(4-methoxyphenyl)-pentan-2-ol (75)

Edukte: 1-(4-Methoxy-2-methylthiophenyl)-2-(4-methoxyphenyl)ethanon (71) Propyljodid
gelbes Öl; Ausbeute: 94%
IR (Film): 3560 cm⁻¹ (m; br; -OH)

### 2-(4-Methoxy-2-methylthiophenyl)-1-(4-methoxyphenyl)-3-phenylpropan-2-ol (76)

Edukte: 1-(4-Methoxy-2-methylthiophenyl)-2-(4-methoxyphenyl)ethanon (71) Benzylchlorid
gelbes Öl; Ausbeute: 44%
IR (Film): 3560 cm⁻¹ (m; br; -OH)

### 2-Hydroxy-2-(4-methoxy-2-methylthiophenyl)-1-(4-methoxyphenyl)-oct-7-en (77)

In diesem Fall wird zur Darstellung des Grignardreagenzes absolutes Tetrahydrofuran als Lösungsmittel verwendet. Als Edukte dienten 6-Brom-1-hexen und 1-(4-Methoxy-2-methylthiophenyl)-2-(4-methoxyphenyl)ethanon (71)
farbloses Öl; Ausbeute: 85%
IR (Film): 3560 cm⁻¹ (m; br; -OH)

### Dehydratisierung der 1-Alkyl-1,2-diarylethanole

Der Alkohol (ca. 5.0 g) wird in 100 ml Toluol gelöst, mit 10.0 g Oxalsäure versetzt und 24 Stunden am Wasserabscheider unter Rückfluß erhitzt. Nach dem Erkalten wird die Oxalsäure abfiltriert und gründlich mit Toluol nachgewaschen. Die organische Phase wird mit Wasser gewaschen, über MgSO₄ getrocknet und anschließend filtriert. Nach dem Entfernen des Lösungsmittels im Vakuum wird mit Dichlormethan/Petrolether 40-60° (1:1, Volumenteile) über Kieselgel 60 chromatographiert. Da bei der Dehydratisierung zwei Möglichkeiten (mit Ausnahme von 72) zur Wasserabspaltung existieren, können bis zu vier Isomere entstehen. Gefunden wurden jedoch nur jeweils zwei Isomere, die sich nicht trennen lassen. Das Bildungsverhältnis der entstandenen Isomeren liegt mit über 50% auf der Seite der gebildeten 1-Alkene.

### 1-(4-Methoxy-2-methylthiophenyl)-2-(4-methoxyphenyl)ethen (78)

Edukt: 1-(4-Methoxy-2-methylthiophenyl)-2-(4-methoxyphenyl)ethanol (72)
farblose Kristalle; Schmp.: 69-70°C Ausbeute: 79%

### 2-(4-Methoxy-2-methylthiophenyl)-1-(4-methoxyphenyl)propen (79)

Edukt: 2-(4-Methoxy-2-methylthiophenyl)-1-(4-methoxyphenyl)propan-2-ol (73)
farblose Kristalle; Schmp.: 55-58°C Ausbeute: 85%

### 2-(4-Methoxy-2-methylthiophenyl)-1-(4-methoxyphenyl)but-1-en (81)

Edukt: 2-(4-Methoxy-2-methylthiophenyl)-1-(4-methoxyphenyl)butan-2-ol (74)
farbloses Öl; Ausbeute: 76%
¹H-NMR (CDCl₃): d = 0.96 - (t;³J=7Hz; 3H,-CH₂CH₃); 2.40 (s;3H,-SCH₃); 2.66 (q; ³J = 7Hz; 2H,-CH₂CH₃); 3.75 (s; 3H, -OCH₃); 3.83 (s; 3H, -OCH₃); 6.36 - 7.16 (m; 7H, ArH); 7.31 (d; ³J = 9Hz; 1H, ArH).

### 2-(4-Methoxy-2-methylthiophenyl)-1-(4-methoxyphenyl)pent-1-en (83)

Edukt: 2-(4-Methoxy-2-methylthiophenyl)-1-(4-methoxyphenyl)pentan-2-ol (75)
gelbes Öl; Ausbeute: 88%
¹H-NMR (CDCl₃): d = 0.86 (t;³J = 7Hz; 3H,-CH₂CH₂CH₃); 1.29-1.75 (m; 2H, -CH₂CH₂CH₃); 2.41 (s; 3H, -SCH₃); 2.34-2.74 (m; 2H, -CH₂CH₂CH₃); 3.75 (s; 3H, -CH₃); 3.83 (s; 3H, -OCH₃); 6.37 - 7.38 (m; 8H, ArH).

### 2-(4-Methoxy-2-methylthiophenyl)-1-(4-methoxyphenyl)-3-phenyl-prop-1-en (85)

Edukt: 2-(4-Methoxy-2-methylthiophenyl)-1-(4-methoxyphenyl)-3-phenylpropan-2-ol (76)
gelbe Kristalle (EtOH); Schmp.: 98-101°C; Ausbeute: 82%

### 2-(4-Methoxy-2-methylthiophenyl)-1-(4-methoxyphenyl)octa-1,7-dien (87)

Edukt: 2-Hydroxy-2-(4-methoxy-2-methylthiophenyl)-1-(4-methoxyphenyl)oct-7-en (77)
farbloses Öl; Ausbeute: 95%
¹H-NMR(CDCl₃): d = 1.22-2.84 (m; 8H, -(CH₂)₄-); 2.40 (s; 3H, -SCH₃); 3.72 (s; - OCH₃); 3.80 (s; 3H, -OCH₃); 4.70-5.05 (m; 2H, -CH₂CH=CH₂); 5.28-6.02 (m; 1H, - CH₂CH=CH₂); 6.26-7.24 (m; 8H, ArH).

### 2-Brom-4-methoxyphenylsulfonylchlorid (133) und 4-Brom-2-methoxyphenylsulfonylchlorid (134)

Eine Lösung von 20.0 g (106.9 mmol) 3-Bromanisol in 100 ml absolutem Dichlormethan wird im Eisbad auf 0°C gekühlt. Anschließend werden 25.0 g (214.0 mmol, 14.2 ml) Chlorsulfonsäure langsam (ca. 45 min.) zugetropft. Eventuell ausfallende Sulfonsäurederivate gehen später wieder in Lösung. Kommt die HCl-Entwicklung zum Erliegen, wird portionsweise Phosphorpentachlorid zugesetzt, bis eine klare homogene Lösung entsteht und keine Steigerung der HCl-Entwicklung erkennbar ist. Man rührt weitere 30 Minuten im Eisbad, gießt auf Eiswasser, trennt die Phase im Scheidetrichter und extrahiert die wäßrige Phasen zweimal mit Dichlormethan. Die vereinigten organischen Phasen werden mit Wasser gewaschen, über MgSO₄ getrocknet, filtriert und im Vakuum eingeengt. DerRückstand wird mittels Säulenchromatographie (Kieselgel 60; Dichlormethan) gereinigt. Bei der Synthese entstehen zwei Isomere, die auf dieser Stufe nicht getrennt werden.
Verbindung 133: 2-Brom-4-methoxyphenylsulfonylchlorid
Verbindung 134: 4-Brom-2-methoxyphenylsulfonylchlorid
Das Bildungsverhältnis hängt stark von den Reaktionsbedingungen ab, wobei die Bildung von Verbindung 133 dominiert. Die nachfolgenden Angaben beziehen sich auf das Isomerengemisch.
farblose Kristalle (EtOH); Schmp.: 79-80°C; Ausbeute: 97%

| | | |
|---|---|---|
| C₇H₆O₃SBrCl (285.5) | Ber.: C 29.44 | H 2.12 |
| | Gef.: C 29.43 | H 1.92 |

### 2-Brom-4-methoxyphenylmercaptan (135) und 4-Brom-2-methoxyphenylmercaptan (136)

Eine Mischung aus 1.12 g (36.2 mmol) rotem Phosphor, 4.5 ml Eisessig und 53.0 mg (0.2 mmol) Jod wird in einem Dreihalskolben mit Rückflußkühler zum leichten Sieden erhitzt. Bei konstant gehaltener Temperatur werden 13.3 mmol des Gemisches aus (133) und (134) portionsweise so zugesetzt, daß ein Entweichen der sich bildenden Joddämpfe vermieden wird. Anschließend wird das Gemisch 2 h unter Rückfluß erhitzt. Man läßt leicht abkühlen, versetzt mit 0.8 ml Wasser und refluxiert erneut 1 h. Nach dem Erkalten wird mit 50 ml Wasser verdünnt und dreimal mit Dichlormethan extrahiert. Die vereinigten organischen Phasen werden mit Wasser säurefrei gewaschen, über MgSO₄ getrocknet, filtriert und im Vakuum eingeengt. Der Rückstand wird mit Petrolether 40-60°C / Essigester (9:1, Volumenteile) über Kieselgel 60 chromatographiert. Die Verbindungen 135 und 136. lassen sich nicht trennen.
farbloses Öl; Sdp: 73-76°C (0.1 mm); Ausbeute: 71 %
IR (Film): 2560 cm⁻¹ (w; -SH)

### 2-Brom-4-methoxyphenylthioacetat (137)

Nebenprodukt bei der Synthese von 135 und 136. Die Ausbeuten schwanken zwischen 0 und 40%.
gelbes Öl;
IR (Film): 1710 cm⁻¹ (s;C = 0)

### 2-Brom-4-methoxyphenyl-methyl-sulfid (138) und 4-Brom-2-methoxyphenyl-methyl-sulfid (139)

Die Synthese erfolgt analog der Arbeitsvorschrift zur Methylierung von Thiophenolen. Als Ausgangsmaterial wird das Gemisch der Verbindungen 135 und 136 verwendet. Das entstandene Thioethergemisch wird mittels Säulenchromatographie (Kieselgel 60) mit Petrolether 40-60°C/Essigester (9:1, Volumenteile) getrennt. Die Ausbeuten hängen vom Bildungsverhältnis der Verbindungen 133 und 134 ab.
1. Fraktion: 4-Brom-2-methoxyphenyl-methyl-sulfid (139)
   farblose Kristalle (EtOH); Schmp. 54-55°C; Ausbeute: 20-54%
2. Fraktion: 2-Brom-4-methoxyphenyl-methyl-sulfid (138)
   farbloses Öl; Sdp.: 81-86°C (0.1 mm); Ausbeute: 40-70%
   1H-NMR (CDCl₃)δ= 2,44 (s;3H, -SCH₃); 3,79 (s;3H,-OCH₃); 6,86 (dd; ³J= 9 Hz; 4 J= 3Hz; 1H, ArH); 7,16 (d, ⁴J = 3 Hz, 1H, ArH); 7,20 (d;³J = 9Hz; 1 H,ArH).

### Darstellung der Alkyl-4-methoxybenzyl-ketone

Apparatur ständig unter Stickstoff halten!
Eine Lösung von 0.3 mol Alkylmagnesiumhalogenid aus 0.3 mol (7.3 g) Magnesiumspänen und 0.3 mol Alkylhalogenid in 200 ml absolutem Ether wird unter kräftigem Rühren (KPG-Rührer) portionsweise mit 0.15 mol (32.8 g) wasserfreiem Cadmiumchlorid versetzt. Man erhitzt 30 Minuten zum Sieden, ersetzt den Rückflußkühler durch eine Destillationsbrücke und destilliert etwa 150 ml Ether ab. Der Rückstand wird mit 250 ml absolutem Benzol versetzt. Unter kräftigem Rühren wird bei Raumtemperatur eine Lösung von 0.2 mol (36.9 g) 4-Methoxyphenylessigsäurechlorid (70) in 70 ml absolutem Benzol langsam zugetropft. Anschließend wird die Mischung 1 Stunde unter Rückfluß erhitzt. Nach dem Abkühlen wird vorsichtig mit 2N Salzsäure hydrolysiert. Die organische Phase wird im Scheidetrichter abgetrennt, zweimal mit gesättigter Natriumhydrogencarbonatlösung und Wasser gewaschen und über MgSO₄ getrocknet. Man entfernt das Trockenmittel durch Filtration, zieht das Lösungsmittel im Vakuum ab und destilliert den Rückstand im Ölpumpenvakuum.

### 1-(4-Methoxyphenyl)butan-2-on (140)

Edukte: 4-Methoxyphenylessigsäurechlorid (70); Ethyljodid
farbloses Öl; Sdp.: 72-76°C (0.1 mm); Ausbeute: 59%
IR (Film): 1720 cm⁻¹ (s; C=O)

### 1-(4-Methoxyphenyl)pentan-2-on (141)

Edukte: 4-Methoxyphenylessigsäurechlorid (70); Propyljodid
hellgelbes Öl; Sdp.: 84-88°C (0.1 mm); Ausbeute: 51%
IR (Film): 1720 cm⁻¹ (s; C=O)

### Darstellung der 1-Alkyl-1,2-diarylethanole

Unter Stickstoffspülung werden 0.5 g (21.4 mmol) Magnesiumspäne durch Erwärmen mit etwas Jod aktiviert (Bildung von Joddämpfen). Anschließend wird eine Lösung von 5.0 g (21.4 mmol) 2-Brom-4-methoxyphenyl-methyl-sulfid (138) in 30 ml absolutem Tetrahydrofuran (THF) langsam zu den aktivierten Magnesiumspänen getropft. Die Reaktion springt unter Aufsieden des Lösungsmittels bei gleichzeitiger Entfärbung der Reaktionslösung an. Das Grignardreagenz wird 1 Stunde unter Rückfluß erhitzt. Nach dem Erkalten tropft man eine Lösung von 24.0 mmol eines entsprechenden Ketons in 20 ml absolutem THF zu, erhitzt erneut 2 Stunden unter Rückfluß und hydrolysiert die erkaltete Lösung mit 2N Salzsäure. Das Hydrolysat wird dreimal mit Ether extrahiert, mit gesättigter Natriumhydrogencarbonatlösung und Wasser gewaschen und über MgSO4 getrocknet. Das Trockenmittel wird durch Filtration entfernt, das Lösungsmittel im Vakuum abgezogen und der Rückstand mit Petrolether 40-60°C/Essigester(3:1, Volumenteile) über Kieselgel 60 chromatographiert. Gelegentlich können bereits durch Wasserabspaltung gebildete Stilbene als Folgeprodukte isoliert werden, die aus Ethanol kristallisieren.

### 2-(5-Methoxy-2-methylthiophenyl)-1-(4-methoxyphenyl)-propan-2-ol (142)

Edukte: 2-Brom-4-methoxyphenyl-methyl-sulfid (138) 4-Methoxyphenylaceton
gelbes Öl; Ausbeute: 27%
IR (Film): 3440 cm⁻¹ (s, br; -OH)

### 2-(5-Methoxy-2-methylthiophenyl)-1-(4-methoxyphenyl)-propen (145)

Nebenprodukt bei der Darstellung von 142.

farblose Kristalle (EtOH); Schmp.: 112-113°C; Ausbeute: 13%

### 2-(5-Methoxy-2-methylthiophenyl)-1-(4-methoxyphenyl)-butan-2-ol (143)

Edukte: 2-Brom-4-methoxyphenyl-methyl-sulfid (138) 1-(4-Methoxyphenyl)butan-2-on (140)
gelbes Öl; Ausbeute: 25%
IR (Film): 3550 cm⁻¹ (m, br; -OH)

### 2-(5-Methoxy-2-methylthiophenyl)-1-(4-methoxyphenyl)but-1-en (147)

Nebenprodukt bei der Darstellung von 143.
farblose Kristalle (MeOH); Schmp.: 108-109°C; Ausbeute: 15%

### 2-(5-Methoxy-2-methylthiophenyl)-1-(4-methoxyphenyl)-pentan-2-ol (144)

Edukte: 2-Brom-4-methoxyphenyl-methyl-sulfid (138) 1-(4-Methoxyphenyl)-pentan-2-on (141)
gelbes Öl; Ausbeute: 46%
IR (Film): 3540 cm⁻¹ (m, br; -OH)

### Dehydratisierung der 1-Alkyl-1,2-diarylethanole

Die Synthese erfolgt analog der Arbeitsvorschrift zur Dehydratisierung von Alkoholen (vor Verbindung (78). Die gebildeten Isomere konnten nicht getrennt werden.

### 2-(5-Methoxy-2-methylthiophenyl)-1-(4-methoxyphenyl)-propen (145)

Edukt: 2-(5-Methoxy-2-methylthiophenyl)-1-(4-methoxyphenyl)-propan-2-ol (142)
Analytische Daten siehe vorstehendes Kapitel "Darstellung der 1-Alkyl-1,2-diarylethanole".

### 2-(5-Methoxy-2-methylthiophenyl)-1-(4-methoxyphenyl)-but-1-en (147)

Edukt: 2-(5-Methoxy-2-methylthiophenyl)-1-(4-methoxyphenyl)-butan-2-ol (143)
Analytische Daten siehe vorne.

### 2-(5-Methoxy-2-methylthiophenyl)-1-(4-methoxyphenyl)-pent-1-en (149)

Edukt: 2-(5-Methoxy-2-methylthiophenyl)-1-(4-methoxyphenyl)pentan-2-ol (144)
gelbes Öl; Ausbeute: 83%
¹H-NMR (CDCl₃): d = 0.87 (t; ³J=7Hz; 3H, -CH₂CH₂CH₃); 1.38-1.82 (m; 2H, -CH₂CH₂CH₃); 2.39 (s; 3H, -SCH₃); 2.52-2.83 (m; 2H, -CH₂CH₂CH₃); 3.77 (s; 3H, OCH₃); 3.80 (s; 3H, -OCH₃); 6.35-7.40 (m; 8H, ArH).

### BEISPIELE

### Etherspaltung und Cyclisierung

Eine Lösung von 4.0 mmol der 1,2-Diarylethanone in 8.0 ml absolutem Dichlormethan werden in einer mit Stickstoff gespülten Apparatur im Eisbad auf 0°C gekühlt und langsam (ca. 10 Minuten) mit einer Lösung von 4.0 g (16.0 mmol; 1.5 ml) Bortribromid in 5.0 ml absolutem Dichlormethan versetzt. Man rührt 1/2 Stunde bei 5-10°C, entfernt das Eisbad und rührt über Nacht bei Raumtemperatur. Anschließend tropft man unter Eiskühlung soviel 10%ige Natriumhydrogencarbonatlösung zu, bis die heftige Reaktion abklingt, versetzt mit 20 ml Essigester und rührt 15 Minuten bei Raumtemperatur. Die Phasen werden im Scheidetrichter getrennt. Die wäßrige Phase wird dreimal mit Wasser extrahiert, die vereinigten organischen Phasen mit Wasser gewaschen und über MgSO₄ getrocknet. Nach dem Abfiltrieren des Trockenmittels wird das Lösungsmittel im Vakuum abgezogen. Der Rückstand wird mit Dichlormethan/Essigester (9:1) über Kieselgel 60 chromatographiert. Die Produkte kristallisieren in der Regel aus heißem Dichlormethan.

### 5.) 3-Ethyl-5-hydroxy-2-(4-hydroxyphenyl)- benzo[b]furan (62a)

Edukt: 2-(2,5-Dimethoxyphenyl)-1-(4-methoxyphenyl)butanon (54a)
farblose Kristalle; Schmp.: 163-164°C; Ausbeute: 30%

### 6.) 3-Ethyl-6-hydroxy-2-(4-hydroxyphenyl)- benzo[b]furan (62b)

Edukt: 2-(2,4-Dimethoxyphenyl)-1-(4-methoxyphenyl)butanon (54b)
beige Kristalle; Schmp.: 125-127°C; Ausbeute: 19%

### 7.) 5-Hydroxy-2-(4-hydroxyphenyl)-3-propylbenzo[b]furan (63a)

Edukt: 2-(2,5-Dimethoxyphenyl)-1-(4-methoxyphenyl)pentanon (55a)
farblose Kristalle; Schmp.: 127-128°C; Ausbeute: 44%

### 8.) 6-Hydroxy-2-(4-hydroxyphenyl)-3-propylbenzo[b]furan (63b)

Edukt: 2-(2,4-Dimethoxyphenyl)-1-(4-methoxyphenyl)pentanon (55b)
farblose Kristalle; Schmp.: 151-152°C; Ausbeute: 23%

### 9.) 3-Butyl-5-hydroxy-2-(4-hydroxyphenyl)benzo[b]furan (64a)

Edukt: 2-(2,5-Dimethoxyphenyl)-1-(4-methoxyphenyl)hexanon (56a)
farblose Kristalle; Schmp.: 124-125°C; Ausbeute: 40%

### 10.) 3-Butyl-6-hydroxy-2-(4-hydroxyphenyl)benzo[b]furan (64b)

Edukt: 2-(2,4-Dimethoxyphenyl)-1-(4-methoxyphenyl)hexanon (56b)
farblose Nadeln; Schmp.: 169-170°C; Ausbeute: 29%

### 11.) 3-(6-Bromhexyl)-5-hydroxy-2-(4-hydroxyphenyl)benzo[b]furan (67a)

Edukt: 8-Brom-2-(2,5-dimethoxyphenyl)-1-(4-methoxyphenyl)octanon (59a)
farblose Kristalle; Schmp.: 146-148°C; unter Zersetzung; Ausbeute: 40%

### 12) 3-(6-Bromhexyl)-6-hydroxy-2-(4-hydroxyphenyl)benzo[b]furan (67b)

Edukt: 8-Brom-2-(2,4-dimethoxyphenyl)-1-(4-methoxyphenyl)octanon (59a)
farblose Kristalle; Schmp.: 89-91°C; Ausbeute: 43%

### Arbeitsvorschrift zur Piperidinsubstitution

In einem 100 ml Rundkolben mit Rückflußkühler werden 0.2 mmol der 3-(6-Bromhexyl)benzo[b]furane 67a und 67b in je 50 ml Piperidin gelöst und 4 Stunden unter Rückfluß erhitzt. Nach dem Erkalten wird überschüssiges Piperidin im Vakuum entfernt. Der Rückstand wird mit Dichlormethan/Ethanol (19:1) über neutrales Aluminiumoxyd der Aktivitätsstufe 2 chromatographiert.

### 13.) 5-Hydroxy-2-(4-hydroxyphenyl)-3-(6-N-piperidylhexyl)benzo[b]furan (68a)

Edukt: 3-(6-Bromhexyl)-5-hydroxy-2-(4-hydroxyphenyl)benzo[b]furan (67a)
hellbeige Kristalle; Schmp.: 190-191°C; Ausbeute: 36%

### 6-Hydroxy-2-(4-hydroxyphenyl)-3-(6-N-piperidylhexyl)benzo[b]furan (68b)

Edukt: 3-(6-Bromhexyl)-6-hydroxy-2-(4-hydroxyphenyl)benzo[b]furan (67b)
hellbeige Kristalle; Schmp.: 115°C unter Zersetzung Ausbeute: 36%

*** Ruwet, A. und Renson, M., Bull.Soc.Chim.Belg., 1970, 79, 593-599**

### Cyclisierung zu Benzo[b]thiophenen ^{*}

14.0 mmol eines 1,2-Diarylalkens werden in 20 ml absolutem Chloroform ge löst und im Eisbad auf 0°C gekühlt. Anschließend werden 14.5 mmol (1.96 g; 1.17 ml) Sulfurylchlorid in 10 ml absolutem Chloroform langsam zugetropft. Nach einstündigem Rühren im Eisbad wird das Lösungsmittel im Vakuum abgezogen, wobei die Temperatur des Wasserbades 40°C nicht übersteigen soll. Der ölige Rückstand wird mit 20 ml absolutem Pyridin versetzt und 1 Stunde unter Rückfluß erhitzt. Nach dem Erkalten der Mischung wird auf Eiswasser gegossen, mit konzentrierter Salzsäure angesäuert und dreimal mit Dichlormethan extrahiert. Die vereinigten organischen Phasen werden zweimal mit Wasser gewaschen, über MgSO₄ getrocknet, filtriert und im Vakuum eingeengt. Der Rückstand wird mit Dichlormethan/Petrolether 40-60°C (1:1, Volumenteile) chromatographiert. Das Produkt kristallisiert in der Regel aus Ethanol. Es wurden zwei Isomere isoliert, da die verwendeten 1,2-Diarylalkene als Isomerengemisch eingesetzt wurden.

### 14.) 6-Methoxy-2-(4-methoxyphenyl)benzo[b]thiophen (89b) (nicht mehr beansprucht)

Edukt: 1-(4-Methoxy-2-methylthiophenyl)-2-(4-methoxyphenyl)-ethen (78)
farblose Kristalle (EtOH); Schmp.: 191-193°C Ausbeute: 82% Lit.: 193-194°C

### 15.)..6-Methoxy-2-(4-methoxyphenyl)-3-methylbenzo[b]thiophen (90b)

Edukt: 2-(4-Methoxy-2-methylthiophenyl)-1-(4-methoxyphenyl)-propen (79)
farblose Nadeln (EtOH); Schmp.: 98-99°C Ausbeute: 41%

### 16.) 3-Ethyl-6-methoxy-2-(4-methoxyphenyl)benzo[b]thiophen (92b)

Edukt: 2-(4-Methoxy-2-methylthiophenyl)-1-(4-methoxyphenyl)but-1-en (81)
farblose Kristalle; Schmp.: 90-91°C Ausbeute: 38%

### 17.) 6-Methoxy-2-(4-methoxyphenyl)-3-propylbenzo[b]thiophen (94b)

Edukt: 2-(4-Methoxy-2-methylthiophenyl)-1-(4-methoxyphenyl)pent-1-en (83)
farblose Nadeln (EtOH); Schmp.: 96-97°C Ausbeute: 41%

### 18.)..3-(Hex-5-en-1-yl)-6-methoxy-2-(4-methoxyphenyl)benzo[b]thiophen (98b)

Edukt: 2-(4-Methoxy-2-methylthiophenyl)-1-(4-methoxyphenyl)octa-1,7-dien (87)
hellgelbes Öl; Ausbeute: 53%

### Etherspaltung

Apparatur mit Stickstoff spülen!
4.0 mmol der zu spaltenden Alkoxyverbindung werden in 6,5 mol absolutem Dichlormethan gelöst und im Eisbad gekühlt. Pro Alkoxygruppe werden 1.05 g (4.2 mmol) bzw. 0.4 ml Bortribromid (99.99 %) in 2 ml absolutem Dichlormethan gelöst und unter Stickstoff langsam (ca. 10 min.) zugetropft. Man rührt ca. 30 min. bei 3-5°C, entfernt das Eisbad und rührt 2 h bei Raumtemperatur. Unter erneuter Eiskühlung wird solange mit Natriumhydrogencarbonatlösung versetzt, bis die heftige Hydrolysereaktion abklingt. Man versetzt mit Essigester, rührt ca. 30 mi. bei Raumtemperatur und trennt die Phasen im Scheidetrichter. Die wäßrige Phase wird noch zweimal mit Essigester extrahiert. Die vereinigten organischen Phasen wäscht man 2 x mit Wasser, trocknet über MgSo₄, filtriert und zieht das Lösungsmittel im Vakuum ab (30-40°C Badtemperatur). Das Rohprodukt wird mittels Säulenchromatographie (Kieselgel 60) gereinigt. Als mobile Phase wird ein Dichlormethan/Essigester-Gemisch (9:1, Volumenteile) verwendet. Das gereinigte Produkt kristallisiert in der Regel aus Dichlormethan unter Zusatz einer geringen Menge Essigester.

### 19.)..6-Hydroxy-2-(4-hydroxyphenyl)benzo[b]thiophen (101b) (nicht mehr beansprucht)

Edukt: 6-Methoxy-2-(4-methoxyphenyl)benzo[b]thiophen (89b)
farblose Kristalle; Schmp.: 252-254°C Ausbeute: 63%

### 20.) 6-Hydroxy-2-(4-hydroxyphenyl)-3-methylbenzo[b]thiophen (102b)

Edukt: 6-Methoxy-2-(4-methoxyphenyl)-3-methylbenzo[b]thiophen (90b)
beige Kristalle; Schmp.: 223-225°C Ausbeute: 36%

### 21.)..3-Ethyl-6-hydroxy-2-(4-hydroxyphenyl)benzo[b]thiophen (104b)

Edukt: 3-Ethyl-6-methoxy-2-(4-methoxyphenyl)benzo[b]thiophen (92b)
farblose Kristalle; Schmp.: 160-161°C Ausbeute: 31%

### 22.) 6-Hydroxy-2-(4-hydroxyphenyl)-3-propylbenzo[b]thiophen (106b)

Edukt: 6-Methoxy-2-(4-methoxyphenyl)-3-propylbenzo[b]thiophen (94b)
farblose Kristalle; Schmp.: 115-117°C Ausbeute: 43%

***Zablocki, J.A. et al., J.Med.Chem., 1987,** **30****, 829-838**

### Hydroborierung mit 9-Borbicyclo[3.3.1]nonan (9-BBN)*

Zu einer Lösung von 1.4 mmol Alken in 5.0 ml absolutem Tetrahydrofuran (THF) werden bei 0°C und unter Stickstoffatmosphäre 17.0 ml einer 0.5M 9-BBN-Lösung (8.4 mmol) in absolutem THF zugetropft. Nach 40minütigem Rühren bei 60°C wird im Eisbad auf 0°C gekühlt, mit 3.0 ml Wasser versetzt und weitere 5 Minuten gerührt. Anschließend setzt man 3.0 ml 3N-Natronlauge zu, rührt erneut 5 Minuten unter Eiskühlung und tropft langsam 3.0 ml 30%ige Wasserstoffperoxidlösung zu. Das Reaktionsgemisch wird 30 Minuten gerührt und anschließend mit 10 ml gesättigter Natriumhydrogencarbonatlösung versetzt. Die Mischung wird dreimal mit Essigester extrahiert, mit Wasser gründlich gewaschen und über MgSO₄ getrocknet. Das Trockenmittel wird durch Filtration entfernt, das Lösungsmittel im Vakuum abgezogen und der Rückstand mit Dichlormethan/Essigester (4:1, Volumenteile) über Kieselgel 60 chromatographiert.

### 23.) 3-(6-Hydroxyhexyl)-6-methoxy-2-(4-methoxyphenyl)benzo[b]thiophen (110b)

Edukt: 3-(Hex-5-en-1-yl)-6-methoxy-2-(4-methoxyphenyl)benzo[b]thiophen (98b)
farbloses Öl; Ausbeute: 73%
IR (Film) 3360 cm⁻¹ (s, br; -OH)

### Methansulfonylierung von Alkoholen * (nicht mehr beansprucht)

Unter Stickstoff werden 2.0 mmol eines primären Alkohols in 20 ml absolutem THF gelöst und mit 20.0 mmol Triethylamin (2.0 g, 2.8 ml) versetzt. Anschließend wird bei Raumtemperatur eine Lösung von 10.0 mmol Methansulfonylchlorid (1.4 g, 1.0 ml) in 5.0 ml absolutem THF langsam zugetropft. Es bildet sich sofort ein weißer Niederschlag. Nach 30minütigem Rühren versetzt man mit 30 ml gesättigter Nathumhydrogencarbonatlösung und extrahiert dreimal mit Essigester. Die vereinigten organischen Phasen werden mit gesättigter Natriumchloridlösung und Wasser gewaschen, über MgSO₄ getrocknet, filtriert und im Vakuum eingeengt. Der Rückstand wird mittels Säulenchromatographie (Kieselgel 60) mit Dichlormethan als mobiler Phase gereinigt. Kristalline Produkte werden aus Ethanol umkristallisiert.

### 24.) 3-(6-Methansulfonyloxyhexyl)-6-methoxy-2-(4-methoxyphenyl)benzo[b]thiophen (113b)

Edukte: 3-(6-Hydroxyphenyl)-6-methoxy-2-(4-methoxyphenyl)benzo[b]thiophen (110b); Methansulfonylchlorid
farbloses Öl; Ausbeute: 76%
IR (Film) 1340 cm^{-1;} 1170 cm ⁻¹ (s, -SO₂O-)

### Substitution mit Piperidin

Eine Lösung von 1.0 mmol Methansulfonat in 4 ml Acetonitril/Triethylamin (1:1, Volumenteile) wird mit 5.0 mmol Piperidin (0.43g, 0.5 ml) versetzt und über Nacht bei Raumtemperatur gerührt. Überschüssiges Piperidin wird zusammen mit dem Lösungsmittel im Vakuum abgezogen. Der Rückstand wird in Essigester aufgenommen, zweimal mit Wasser gewaschen und über MgSO₄ getrocknet. Das Trockenmittel wird durch Filtration entfernt, das Lösungsmittel im Vakuum abgezogen und der Rückstand mit Essigester/Triethylamin (30:1, Volumenteile) über Kieselgel 60 chromatographiert.

### 25.) 6-Methoxy-2-(4-methoxyphenyl)-3-(6-N-peperidinylhexyl)benzo[b]thiophen (116b)

Edukte: 3-(6-Methansulfonyloxyhexyl)-6-methoxy-2-(4-methoxyphenyl)benzo[b]thiophen (113b); Piperidin
gelbes Öl; Ausbeute: 60%
¹H-NMR (CDCl₃): d = 1.10-1.80 (m; 14H, -(CH₂)₇-); 2.10-2.45 (m; 6H, -N(CH₂)₃; 2.80 (t; ³J = 7Hz; 2H, =CCH₂CH₂); 3.84 (s; 3H, -OCH₃); 3.86 (s; 3H, -OCH₃); 7.01 (dd; ³J = 9Hz; ⁴J = 2Hz; 1H, ArH); 7.31 (d; ⁴J = 2Hz; 1H, ArH); 7.61 (d; ³J = 9Hz; 1H, ArH); 6.97, 7.44 (AA 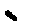 BB 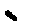 ; ³J = 9Hz; 4H, ArH).

### Etherspaltung

Die Etherspaltung erfolgt analog der allgemeinen Vorschrift vor Verbindung 101b. Als Lösungsmittel wird die 20-fache Menge an absolutem Dichlormethan verwendet. Die Rohprodukte werden mit Essigester/Triethylamin/Ethanol-Gemischen über Kieselgel 60 chromatographiert.Die Produkte kristallisieren aus Dichlormethan/Essigester unter Zusatz von Hexan.

### 26.) 6-Hydroxy-2-(4-hydroxyphenyl)-3-(6-N-piperidinylhexyl)-benzo[b]thiophen (119b)

Edukt: 6-Methoxy-2-(4-methoxyphenyl)-3-(6-N-piperidinylhexyl)benzo[b]thiophen (116b);
Flußmittel für die Säulenchromatographie: Essigester/NEt₃/EtOH (10/10/1, V/V/V)
beige Kristalle; Schmp.: 115°C unter Zersetzung Ausbeute: 46%

*** Ruwet, A. und Renson, M., Bull.Soc.Chim.Belg., 1970, 79, 593-599**

### Cyclisierung der 1,2-Diarylalkene *

Die Synthese wird nach der allgemeinen Vorschrift "Cyclisierung zu Benzo[b]-thiophen" durchgeführt. Es werden 2 Isomere isoliert, da die verwendeten 1,2-Diarylalkene als Isomerengemische eingesetzt wurden. Als Nebenprodukte treten weitere Benzol[b]thiophenderivate auf, die am C4-Atom zusätzlich chloriert sind.

### 27.) 5-Methoxy-2-(4-methoxyphenyl)-3-methylbenzo[b]thiophen (90a)

Edukt: 2-(5-Methoxy-2-methylthiophenyl)-1-(4-methoxyphenyl)propen (145)
farblose Nadeln (EtOH); Schmp.: 106-107°C; Au10sbeute: 41%

### 28.) 3-Ethyl-5-methoxy-2-(4-methoxyphenyl)benzo[b]thiophen (92a)

Edukt: 2-(5-Methoxy-2-methylthiophenyl)-1-(4-methoxyphenyl)but-1-en (147)
gelbe Kristalle (EtOH); Schmp.: 116-117°C; Ausbeute: 48%

### 29.) 5-Methoxy-2-(4-methoxyphenyl)-3-propylbenzo[b]thiophen (94a)

Edukt: 2-(5-Methoxy-2-methylthiophenyl)-1-(4-methoxyphenyl)pent-1-en (149)
farblose Kristalle (EtOH); Schmp.: 76-77°C; Ausbeute: 47%

### Etherspaltung

Die Etherspaltung erfolgt analog der allgemeinen Vorschrift. Die Rohprodukte werden mittels Säulenchromatographie (Kieselgel 60) mit Dichlormethan/Essigester (9:1, Volumenteile) als mobiler Phase gereinigt. Die Produkte kristallisieren aus heißem Dichlormethan.

### 30.) 5-Hydroxy-2-(4-hydroxyphenyl)-3-methylbenzo[b]thiophen (102a)

Edukt: 5-Methoxy-2-(4-methoxyphenyl)-3-methylbenzo[b]thiophen (90a)
farblose Kristalle; Schmp.: 204-205°C; Ausbeute: 71%

### 31.) 3-Ethyl-5-hydroxy-2-(4-hydroxyphenyl)benzo[b]thiophen (104a)

Edukt: 3-Ethyl-5-methoxy-2-(4-methoxyphenyl)benzo[b]thiophen (92a)
farblose Kristalle; Schmp.: 173-174°C; Ausbeute: 78%

### 32.) 5-Hydroxy-2-(4-hydroxyphenyl)-3-propylbenzo[b]thiophen (106a)

Edukt: 5-Methoxy-2-(4-methoxyphenyl)-3-propylbenzo[b]thiophen (94a)
farblose Kristalle; Schmp.: 152-153°C; Ausbeute: 81%

### Einführung einer Seitenkette in 2-Phenylbenzo[b]thiophene

### a) 3-(6-Bromhexanoyl)-6-methoxy-2-(4-methoxyphenyl)benzo[b]thiophen

Eine Lösung von 1,09 g (5,13 mmol) 6-Bromhexansäurechlorid in 50 ml 1,2-Dichlorethan wird mit 1,40 g (5,13 mmol) 6-Methoxy-2-(4-methoxyphenyl)benzo[b]thiophen¹ versetzt; anschließend werden 0,80 g (6,15 mmol) AlCl₃ in drei Portionen unter Rühren bei Raumtemperatur zugegeben. Nachdem man noch 1 Stunde gerührt hat, gießt man in Eiswasser und extrahiert mit Ether. Nach dem Waschen mit gesättigter NaCℓ-Lösung und Trocknen (MgSO₄) zieht man das Solvens ab und reinigt den Rücktstand durch Säulenchromatographie (SiO₂; CH₂Cℓ₂/Petrolether 3:1). Man erhält ein gelbes Öl in einer Ausbeute von 55 %.
¹ Jones CD, Jevmikov MG, Pike AJ, Peters MK, Black LJ, Thompson AR, Falcone JF, Clemens JA; J.Med.Chem. 1984, **27**, 1057-1066

### b) 3-(6-Bromhexyl)6-methoxy-2-(4-methoxyphenyl)benzo[b]thiophen

Unter Stickstoff werden 46,0 mg (1,21 mmol) LiAℓH₄ in 1 ml trockenem Ether vorgelegt. Nach dem Abkühlen im Eisbad werden 161.6 mg (1,21 mmol) AlCl₃ in 1ml trockenem Ether zugetropft. Nach einer Minute wird das Eisbad entfernt und 542,0 mg (1,21 mmol) 3-(6-Bromhexanoyl)-6-methoxy-2-(4-methoxyphenyl)benzo[b]thiophen in 2 ml trockenem Ether unter schwachem Sieden zugetropft. Nach 30 Minuten werden nacheinander unter Eiskühlung 2 ml Wasser und 2 ml 6N H₂SO₄ zugetropft. Die Mischung wird mit Ether extrahiert. Nach dem Waschen mit Wasser und Trocknen wird der Ether abgezogen und das Produkt aus n-Hexan umkristallisiert.
farblose Kristalle, Schmelzpunkt: 93-95°C, Ausbeute: 80 %

## Patentansprüche

1. 2-Phenylbenzo[b]furane und -thiophene der allgemeinen Formel I 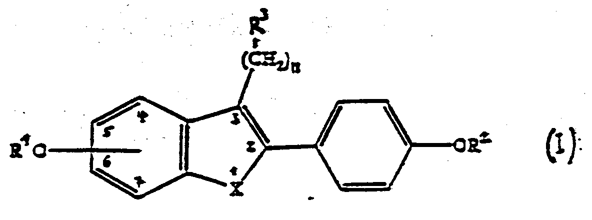
worin R¹ und R² unabhängig voneinander ein Wasserstoffatom, eine Alkylgruppe mit 1 bis 10 Kohlenstoffatomen, eine Benzylgruppe, eine Gruppe -C(O)R⁴, wobei R⁴ eine Alkyl- oder Alkyloxygruppe mit 1 bis 10 Koblenstoffatomen oder ein Phenylrest ist, oder eine Carbamoylgruppe -C(O)NR⁵R⁶, wobei R⁵ und R⁶ unabhängig voneinander ein Wasserstoffatom oder eine Alkylgruppe mit 1 bis 10 Kohlenstoffatomen ist,
X ein Sauerstoff- oder Schwefelatom und n eine ganze Zahl von 4 bis 12, wenn R³ eine Aminogruppe -NR⁷R⁸ ist, wobei R⁷ und R⁸ unabhängig voneinander für ein Wasserstoffatom oder eine Alkylgruppen mit 1 bis 10 Kohlenstoffatomen oder R⁷-R⁸ gemeinsam für eine Alkylgruppe -(CH₂)ₘ-, wobei m 2, 3, 4, 5 oder 6 ist, oder die Gruppe -(CH₂)₂O(CH₂)₂- stehen, oder
X ein Sauerstoff- oder Schwefelatom und n eine ganze Zahl von 0 bis 12 wenn R³ ein Wasserstoffatom ist, wobei n nicht 0 sein kann, wenn X ein Schwefelatom ist und wobei n nicht 0 oder 1 sein kann, wenn X ein Sauerstoffatom ist,
bedeuten.

2. 3-Ethyl-5-hydroxy-2-(4-hydroxyphenyl)benzo[b]furan
3-Ethyl-6-hydroxy-2-(4-hydroxyphenyl)benzo[b]furan
5-Hydroxy-2-(4-hydroxyphenyl)-3-propylbenzo[b]furan
6-Hydroxy-2-(4-hydroxyphenyl)-3-propylbenzo[b]furan
3-Butyl-5-hydroxy-2-(4-hydroxyphenyl)benzo[b]furan
3-Butyl-6-hydroxy-2-(4-hydroxyphenyl)benzo[b]furan
5-Hydroxy-2-(4-hydroxyphenyl)-3-(6-N-piperidylhexyl)-benzo[b]furan
6-Hydroxy-2-(4-hydroxyphenyl)-3-(6-N-piperidylhexyl)-benzo[b]furan
6-Hydroxy-2-(4-hydroxyphenyl)-3-methylbenzo[b]thiophen
3-Ethyl-6-hydroxy-2-(4-hydroxyphenyl)benzo[b]thiophen
6-Hydroxy-2-(4-hydroxyphenyl)-3-propylbenzo[b]thiophen
6-Hydroxy-2-(4-hydroxyphenyl)-3-(6-N-piperidylhexyl)-benzo[b]thiophen

3. Verfahren zur Herstellung von 2-Phenylbenzo[b]furanen und -thiophenen der allgemeinen Formel I, dadurch gekennzeichnet, daß
a) wenn X letztendlich Sauerstoff sein soll, eine Verbindung der allgemeinen Formel IIa 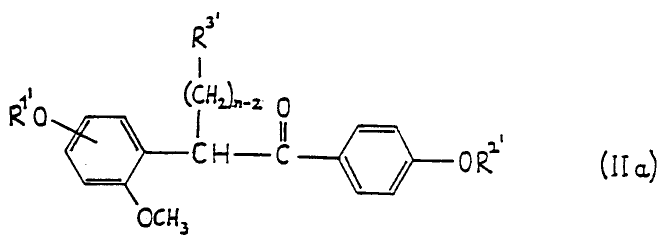 worin
R^{1'} und R^{2'} unabhängig voneinander je eine Alkylgruppe mit 1 bis 4 Kohlenstoffatomen sowie
R^{3'} entweder -(CH₂)₂-R, -(CH₂)₂-Hal, wobei Hal ein Halogenatom und insbesondere ein Bromatom ist einen Vinylrest -CH=CH₂ oder einen Rest -CH₂-C(O)NR⁷R⁸
bedeuten
mit einer Lewis-Säure unter Spaltung der Ethergruppierungen zu einer Verbindung der allgemeinen Formel Ia 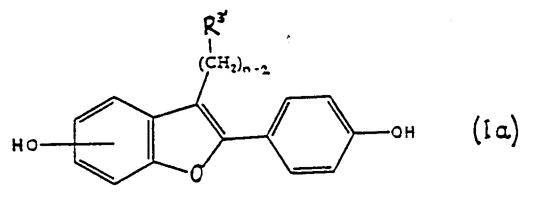 cyclisiert und wenn R^{3'}-(CH₂)₂-Hal bedeutet, Hal durch Umsetzung der Verbindung der Formel Ia mit einem primären, sekundären oder cyclischen Amin der Formel HNR⁷R⁸ gegen den entsprechenden Aminrest -NR⁷R⁸ ausgetauscht oder wenn R^{3'} einen Vinylrest bedeutet, durch endständige Hydroxylierung der vinylischen Doppelbindung, Überführung der erzeugten Hydroxygruppe in eine bessere Abgangsgruppe und diese analog zum Fall, daß R^{3'} ein Halogenatom bedeutet gegen einen Aminrest -NR⁷R⁸ ausgetgauscht oder wenn R^{3'} den Rest -CH₂-C(O)NR⁷R⁸ bedeutet, die Carbonylgruppe mit Lithium-aluminiumhydrid vollständig reduziert und die freien Hydroxygruppen gegebenenfalls verethert oder verestert, oder
b) wenn X letztendlich Schwefel sein soll, eine Verbindung der allgemeinen Formel IIb 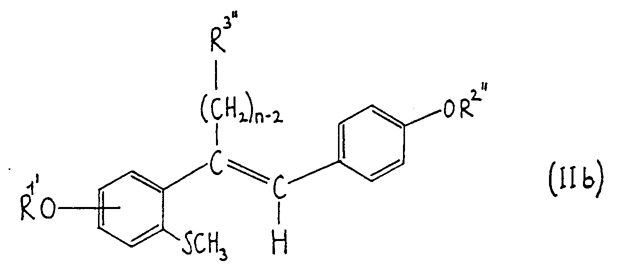 worin
R^{1'} und R^{2'} dieselbe Bedeutung wie in Formel IIa haben und R^{3''}-(CH₂)₂-R, - (CH₂)₂- Hal ein Halogenatom und insbesondere ein Bromatom ist, oder einen Vinylrest -CH=CH₂
bedeutet,
mit einem Gemisch aus Sulfurylchlorid und Pyridin zu dem entsprechenden Benzo[b]thiophenderivat der allgemeinen Formel Ib 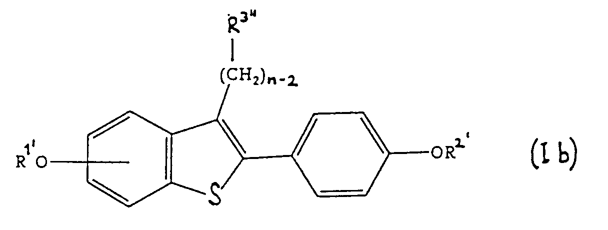 cyclisiert und dann wenn R^{3''}-(CH₂₎₂-Hal oder einen Vinylrest -CH=CH₂ bedeutet wie unter a) für diese beiden Fälle angegeben die Verbindung der allgemeinen Formel Ib weiter umgesetzt wird und anschließend mit einer Lewis-Säure die Ethergruppierungen gegebenenfalls gespalten und die freien Hydroxygruppen gegebenenfalls verethert oder verestert werden, oder
c) wenn X letztendlich Schwefel sein soll, eine Verbindung der allgemeinen Formel II c 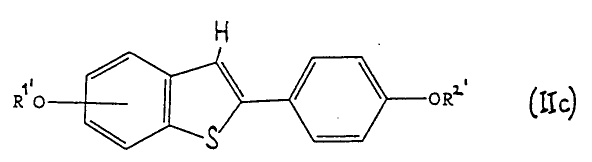 worin R^{1'} und R^{2'} dieselbe Bedeutung wie in Formel II a haben,
mit einem Säurehalogenid der allgemeinen Formel 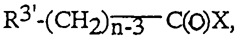 wobei R^{3'} und n dieselbe Bedeutung wie in Formel II a haben und X ein Chlor- oder Bromatom ist, acyliert und nachfolgend mit LiAlH₄/AlCl₃ zu einer Verbindung der allgemeinen Formel I c 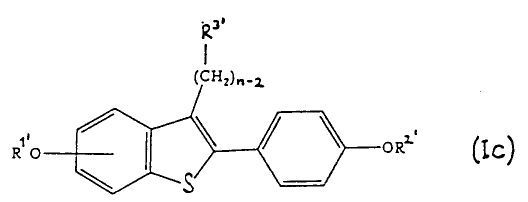 reduziert und wie unter a) beschrieben weiter umgesetzt wird.

4. Pharmazeutische Präparate, dadurch gekennzeichnet, daß sie mindestens eine Verbindung gemäß Anspruch 1 oder 2 sowie einen inerten, pharmazeutisch verträglichen Träger enthalten.

5. Verwendung der Verbindungen gemäß Anspruch 1 oder 2 zur Herstellung von Arzneimitteln.

## Claims

1. 2-Phenylbenzo[b]-furans and -thiophenes of the general formula I 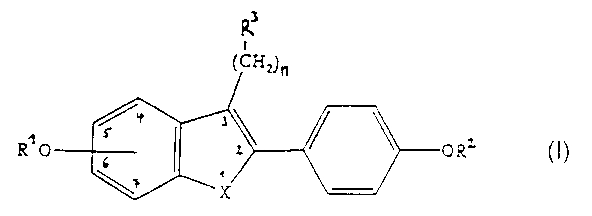 wherein
R¹ and R² are each independently of the other a hydrogen atom an alkyl group having from 1 to 10 carbon atoms, a benzyl group, a group -C(O)R⁴ wherein R⁴ is an alkyl or alkyloxy group having from 1 to 10 carbon atoms or a phenyl radical, or a carbamoyl group -C(O)NR⁵R⁶ wherein R⁵ and R⁶ are each independently of the other a hydrogen atom or an alkyl group having from 1 to 10 carbon atoms,
X is an oxygen or sulphur atom and n is an integer from 4 to 12 when R³ is an amino group -NR⁷R⁸ wherein R⁷ and R⁸ are each independently of the other a hydrogen atom or an alkyl group having from 1 to 10 carbon atoms or R⁷-R⁸ together are an alkylene group -(CH₂)ₘ-, wherein m is 2, 3, 4, 5 or 6, or the group -(CH₂)₂O(CH₂)₂-, or
X is an oxygen or sulphur atom and n is an integer from 0 to 12 when R³ is a hydrogen atom, but n cannot be 0 when X is a sulphur atom and n cannot be 0 or 1 when X is an oxygen atom.

2. 3-Ethyl-5-hydroxy-2-(4-hydroxyphenyl)benzo[b]furan,
3-ethyl-6-hydroxy-2-(4-hydroxyphenyl)benzo[b]furan,
5-hydroxy-2-(4-hydroxyphenyl)-3-propylbenzo[b]furan,
6-hydroxy-2-(4-hydroxyphenyl)-3-propylbenzo[b]furan,
3-butyl-5-hydroxy-2-(4-hydroxyphenyl)benzo[b]furan,
3-butyl-6-hydroxy-2-(4-hydroxyphenyl)benzo[b]furan,
5-hydroxy-2-(4-hydroxyphenyl)-3-(6-N-peperidylhexyl)-benzo[b]furan,
6-hydroxy-2-(4-hydroxyphenyl)-3-(6-N-piperidylhexyl)-benzo[b]furan,
6-hydroxy-2-(4-hydroxyphenyl)-3-methylbenzo[b]thiophene,
3-ethyl-6-hydroxy-2-(4-hydroxyphenyl)benzo[b]thiophene,
6-hydroxy-2-(4-hydroxyphenyl)-3-propylbenzo[b]thiophene,
6-hydroxy-2-(4-hydroxyphenyl)-3-(6-N-piperidinylnexyl)-benzo[b]thiophene.

3. Process for the preparation of 2-phenylbenzo[b]furans and -thiophenes of the general formula I, characterised in that
a) when X is ultimately to be oxygen, a compound of the general formula IIa 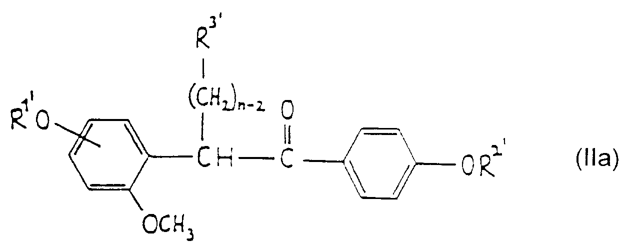 wherein
R^{1'} and R^{2'} are each independently of the other an alkyl group having from 1 to 4 carbon atoms, and
R^{3'} is either -(CH₂)₂-R, -(CH₂)₂-Hal, wherein Hal is a halogen atom and especially a bromine atom, a vinyl radical -CH=CH₂ or a radical -CH₂-C(O)NR⁷R⁸,
is cyclised with a Lewis acid, with cleavage of the ether groupings, to form a compound of the general formula Ia 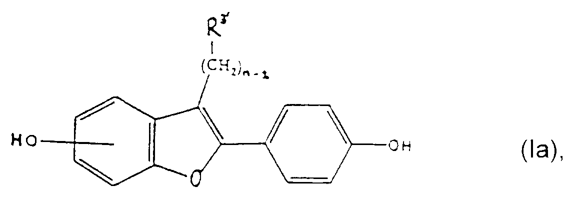 and when R^{3'} is -(CH₂)₂-Hal, by reaction of the compound of formula Ia with a primary, secondary or cyclic amine of the formula HNR⁷R⁸, Hal is replaced by the corresponding amine radical -NR⁷R⁸, or when R^{3'} is a vinyl radical, terminal hydroxylation of the vinylic double bond is carried out and the hydroxy group produced is converted into a better leaving group and that group is replaced by an amine radical -NR⁷R⁸ analogously to the case where R^{3'} is a halogen atom, or when R^{3'} is the radical -CH₂-C(O)NR⁷R⁸, the carbonyl group is completely reduced with lithium aluminium hydride and the free hydroxy groups are optionally etherifed or esterified, or
b) when X is ultimately to be sulphur, a compound of the general formula IIb 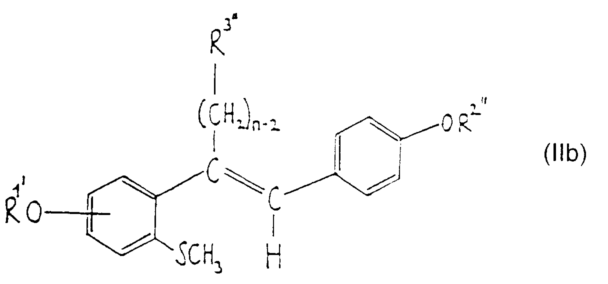 wherein
R^{1'} and R^{2'} have the same meanings as in formula IIa and R^{3''} is -(CH₂)₂-R, -(CH₂)₂-Hal, wherein Hal is a halogen atom and especially a bromine atom, or a vinyl radical -CH=CH₂,
is cyclised with a mixture of sulphuryl chloride and pyridine to form the corresponding benzo[b]thiophene derivative of the general formula Ib 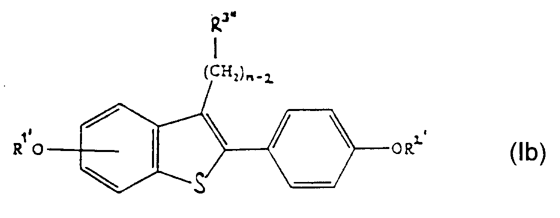 and then when R^{3''} is -(CH₂)₂-Hal or a vinyl radical -CH=CH₂, the compound of the general formula Ib is further reacted as indicated for those two cases under a) and then the ether groupings are optionally cleaved with a Lewis acid and the free hydroxy groups are optionally etherified or esterified, or
c) when X is ultimately to be sulphur, a compound of the general formula IIc 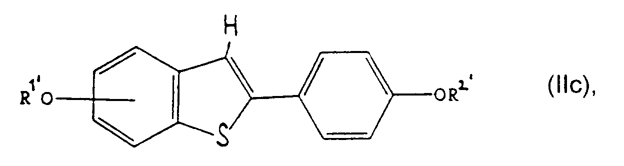 wherein R^{1'} and R^{2'} have the same meanings as in formula IIa,
is acylated with an acid halide of the general formula 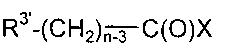 wherein R^{3'} and n have the same meanings as in formula IIa and X is a chlorine or bromine atom, and then reduced with LiAlH₄/AlCl₃ to form a compound of the general formula Ic 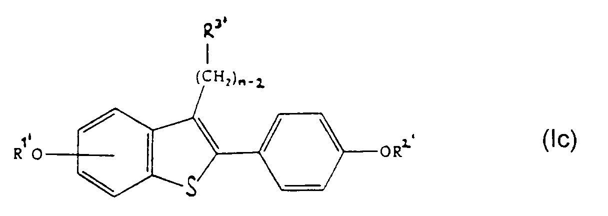 and is reacted further as described under a).

4. Pharmaceutical preparations, characterised in that they contain at least one compound according to claim 1 or 2 and an inert, pharmaceutically acceptable carrier.

5. The use of the compounds according to claim 1 or 2 in the preparation of medicaments.

## Revendications

1. 2-Phénylbenzo[b]furannes et -thiophènes de formule générale I : 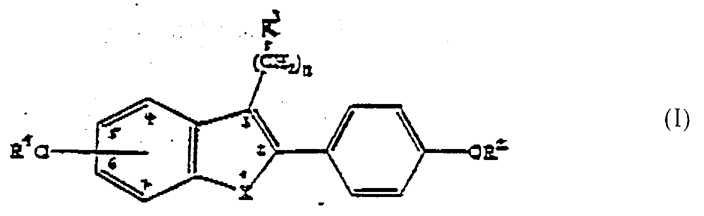
dans laquelle R¹ et R² sont, indépendamment l'un de l'autre, un atome d'hydrogène, un groupe alkyle ayant 1 à 10 atomes de carbone, un groupe benzyle, un groupe -C(O)R⁴, R⁴ étant un groupe alkyle ou alkyloxy avec 1 à 10 atomes de carbone ou un radical phényle, ou un groupe carbamoyle -C(O)NR⁵R⁶, dans lequel R⁵ et R⁶ sont, indépendamment l'un de l'autre, un atome d'hydrogène ou un groupe alkyle ayant 1 à 10 atomes de carbone, X est un atome d'oxygène ou de soufre et n est un nombre entier de 4 à 12. lorsque R³ est un groupe amino -NR⁷R⁸, R⁷ et R⁸ étant, indépendamment l'un de l'autre, un atome d'hydrogène ou un groupe alkyle ayant 1 à 10 atomes de carbone ou bien R⁷-R⁸ forment ensemble un groupe alkylène -(CH₂)ₘ-, dans lequel m est égal à 2, 3, 4, 5 ou 6, ou bien le groupe -(CH₂)₂O(CH₂)₂-, ou
X est un atome d'hydrogène ou de soufre et n est un nombre entier de 0 à 12, lorsque R³ est un atome d'oxygène, n ne pouvant être égal à 0 lorsque X est un atome de soufre et n ne pouvant être égal à 0 ou à 1 lorsque X est un atome d'oxygène.

2. 3-Ethyl-5-hydroxy-2-(4-hydroxyphényl) benzo[b]furanne
3-Ethyl-6-hydroxy-2-(4-hydroxyphényl)benzo[b]furanne
5-Hydroxy-2-(4-hydroxyphényl)-3-propylbenzo[b]furanne
6-Hydroxy-2-(4-hydroxyphényl)-3-propylbenzo[b]furanne
3-Butyl-5-hydroxy-2-(4-hydroxyphényl)benzo[b]furanne
3-Butyl-6-hydroxy-2-(4-hydroxyphényl)benzo[b]furanne
5-Hydroxy-2-(4-hydroxyphényl)-3-(6-N-pipéridylhexyl)-benzo[b]furanne
6-Hydroxy-2-(4-hydroxyphényl)-3-(6-N-piperidylhexyl)-benzo[b]furanne
6-Hydroxy-2-(4-hydroxyphényl)-3-méthylbenzo[b]thiophène
3-Ethyl-6-hydroxy-2-(4-hydroxyphényl)benzo[b]thiophène
6-Hydroxy-2-(4-hydroxyphényl)-3-propylbenzo[b]thiophène
6-Hydroxy-2-(4-hydroxyphényl)-3-(6-N-piperidinylhexyl)-benzo[b]thiophene.

3. Procédé de préparation de 2-phénylbenzo[b] furannes et -thiophènes de formule générale (I), caractérisé en ce que
a) lorsque X doit être finalement de l'oxygène, un composé de formule générale IIa : 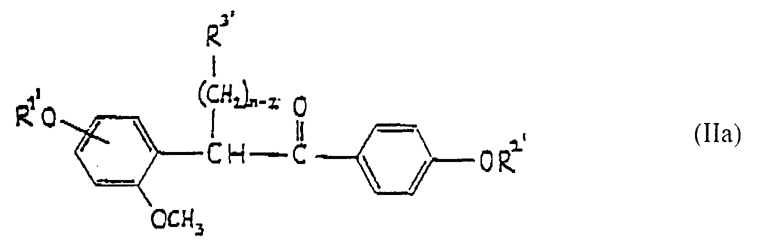 dans laquelle
R^{1'} et R^{2'} sont chacun, indépendamment l'un de l'autre, un groupe alkyle ayant 1 à 4 atomes de carbone, et
R^{3'} est -(CH₂)₂-R, -(CH₂)₂-Hal, où Hal est un atome d'halogène et, en particulier, un atome de brome, un radical vinyle -CH=CH₂ ou un radical -CH₂-C(O)NR⁷R⁸,
est cyclisé avec un acide de Lewis pour former, par dissociation des groupes éthers, un composé de formule générale Ia : 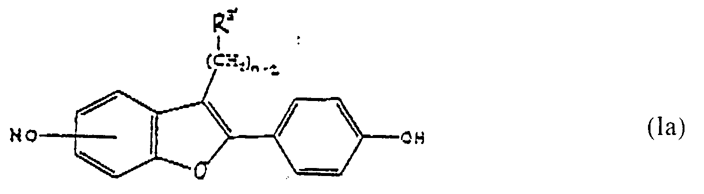 et, lorsque R^{3'} est -(CH₂)₂-Hal le groupe Hal est par réaction du composé de formule Ia avec une amine primaire, secondaire ou cyclique de formule HNR⁷R⁸, échangé par le radical -NR⁷R⁸ correspondant, ou, lorsque R^{3'} est un radical vinyle, le groupe Hal est par hydroxylation en fin de chaîne de la double liaison vinylique, transfert du groupe hydroxyle obtenu dans un meilleur groupe initial et ce, de manière analogue au cas où R^{3'} est un atome d'halogène, échangé par un radical amine -NR⁷R⁸, ou lorsque R^{3'} est le radical -CH₂-C(O)NR⁷R⁸, le groupe carbonyle est complètement réduit avec un hydrure de lithium et d'aluminium et les groupes hydroxyles libres sont éventuellement éthérifiés ou estérifiés, ou bien
b) lorsque X doit être finalement un atome de soufre, un composé de formule générale IIb : 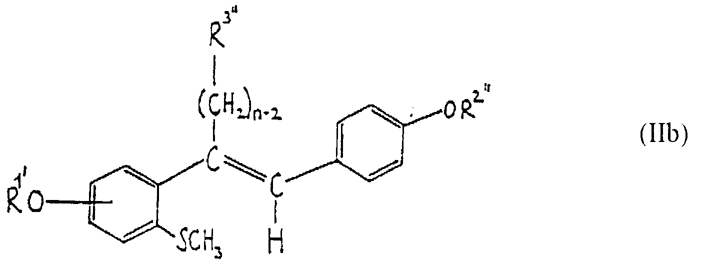 dans laquelle
R^{1'} et R^{2'} ont la même signification que dans la formule IIa et R^{3''} est -(CH₂)₂-R, -(CH₂)₂-Hal, Hal étant un atome d'halogène et, en particulier, un atome de brome, ou un radical vinyle -CH=CH₂,
est cyclisé avec un mélange de chlorure de sulfuryle et de pyridine pour former le dérivé correspondant de benzo[b]thiophène de formule générale Ib : 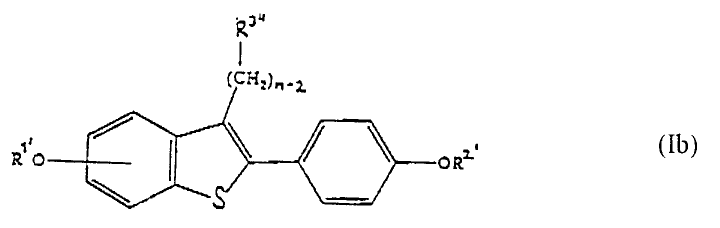 et, lorsque R^{3''} est -(CH₂)₂-Hal ou un radical vinyle -CH=CH₂, on fait réagir le composé de formule générale Ib comme indiqué sous a) pour ces deux cas et, ensuite, les groupes éthers sont , au moyen d'un acide de Lewis, éventuellement dissociés et les groupes hydroxyles libres sont éventuellement éthérifiés ou estérifiés, ou bien
c) lorsque X doit finalement être un atome de soufre, un composé de formule générale IIc : 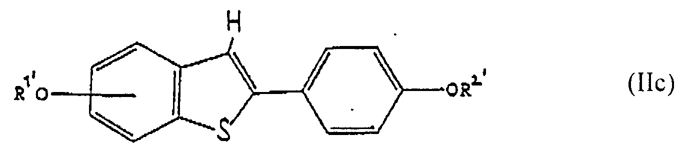 dans laquelle R^{1'} et R^{2'} ont la même signification que dans la formule IIa, est acylé avec un halogénure d'acide de formule générale R^{3'}-(CH₂)ₙ₋₃-C(O)X, où R^{3'} et n ont la même signification que dans la formule IIa et X est un atome de chlore ou de brome, et est ensuite réduit avec du LiAlH₄/AlCl₃ pour obtenir un composé de formule générale Ic : 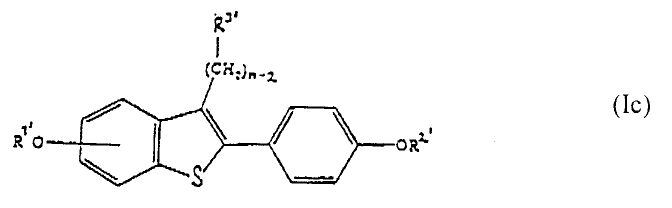 et on poursuit la réaction comme décrit sous a).

4. Préparations pharmaceutiques, caractérisées en ce qu'elles contiennent au moins un composé selon la revendication 1 ou 2 ainsi qu'un support inerte pharmaceutiquement compatible.

5. Utilisation des composés selon la revendication 1 ou 2 pour la préparation de médicaments.
